# EUROPÄISCHE PATENTSCHRIFT

(11) **EP 0 696 289 B1**
(45) Veröffentlichungstag und Bekanntmachung des Hinweises auf die Patenterteilung: **12.03.1997**
(21) Anmeldenummer: 94914315.0
(22) Anmeldetag: 28.04.1994
(51) Int. Cl.: C07F 9/6509, A61K 31/66, C07D 241/44, A61K 31/495, C07D 403/06, C07F 9/6558

(54) **BENZO[F]CHINOXALINDIONDERIVATIVE, DEREN HERSTELLUNG UND VERWENDUNG IN ARZNEIMITTELN**
BENZO[F]QUINOXALINDIONE DERIVATIVES, THEIR PREPARATION AND THEIR USE IN DRUGS
DERIVES DE BENZO[F]QUINOXALINEDIONE, LEUR FABRICATION ET LEUR UTILISATION DANS DES MEDICAMENTS

(30) Priorität: 28.04.1993 DE 4314592
(43) Veröffentlichungstag der Anmeldung: 14.02.1996
(73) Patentinhaber: SCHERING AKTIENGESELLSCHAFT, 13353 Berlin (DE)
(72) Erfinder: HÖLSCHER, Peter, D-13357 Berlin (DE); TURSKI, Lechoslaw, D-13505 Berlin (DE)
(86) Internationale Anmeldenummer: DE9400495
(87) Internationale Veröffentlichungsnummer: WO9425470

(56) Entgegenhaltungen:
- EP-A- 0 511 152
- WO-A-91/13878
- WO-A-93/08173

## Beschreibung

Die Erfindung betrifft Benzo[f]chinoxalindionderivate, deren Herstellung und Verwendung in Arzneimitteln.

Es ist beispielsweise aus EP-A-511152 bekannt, daß Chinoxalinderivate Affinität an die Quisqualat-Rezeptoren besitzen und sich auf Grund dieser Rezeptorbindung als Arzneimittel zur Behandlung von Krankheiten des zentralen Nervensystems eignen.

Die erfindungsgemäßen Verbindungen haben die Formel I 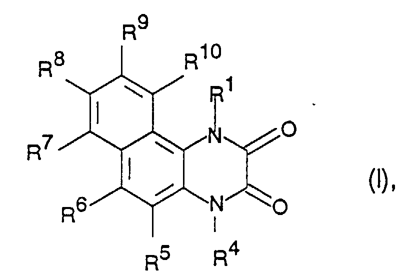 worin
R¹ und R⁴ gleich oder verschieden sind und Wasserstoff, mit R² substituiertes C₁₋₁₂-Alkyl, mit R² substituiertes C₂₋₁₂-Alkenyl, mit R² substituiertes C₂₋₁₂-Alkinyl, mit R² substituiertes C₃₋₇-Cycloalkyl, -(CH₂)ₙ-C₆₋₁₂-Aryl, das im Aryl- oder im Alkylrest mit R² substituiert ist oder -(CH₂)ₙ- Hetaryl, das im Hetaryl- oder Alkylrest mit R² substituiert ist und R¹ und R⁴ nicht gleichzeitig Wasserstoff bedeuten,
R⁵, R⁶, R⁷, R⁸, R⁹ und R¹⁰ gleich oder verschieden sind und Wasserstoff, Halogen, Nitro, NR¹⁶R¹⁷, NHCOR¹¹, SO₀₋₃R¹², C₃₋₇-Cycloalkyloxy, COR¹³, Cyano, CF₃, OCH₂CF₃,C₁₋₆- Alkyl oder C₁₋₄-Alkoxy,
wobei
R² -CN,-Tetrazol,-C(NOH)NH2,-CO-R³ oder -PO-XY und R² ein- bis zweifach gleich oder verschieden steht und
n 0, 1, 2, 3, 4 oder 5 ist,
R³ Hydroxy, C₁₋₆-Alkoxy oder NR¹⁴R¹⁵,
X und Y gleich oder verschieden sind und Hydroxy, C₁₋₆-Alkoxy, -O-(CH₂)ₚ-O-, C₁₋₄-Alkyl oder NR¹⁴R¹⁵ bedeuten und p 1, 2 oder 3 ist und
R¹¹ C₁₋₆-Alkyl oder Phenyl, das mit Halogen substituiert sein kann,
R¹² Wasserstoff, C₁₋₄-Alkyl, NH₂, N(C₁₋₄-Alkyl)₂, -NH(C₁₋₄-Alkyl), -NH-CH₂CONH₂, -CH₂CONH₂, CF₃ oder -NH-(CH₂)ₙ-R² und
R¹³ Hydroxy, C₁₋₆-Alkoxy, C₁₋₆-Alkyl oder NR¹⁴R¹⁵ bedeuten
R¹⁴ und R¹⁵, R¹⁶ und R¹⁷ gleich oder verschieden sind und Wasserstoff, -CO-C₁₋₆-Alkyl, Phenyl oder C₁₋₆-Alkyl, das gegebenenfalls mit C₁₋₄-Alkoxy oder einer gegebenenfalls mit C₁₋₄-Alkyl mono- oder di-substituierter Aminogruppe substituiert sein kann, oder gemeinsam mit dem Stickstoffatom einen 5-7-gliedrigen gesättigten Heterocyclus bilden, der ein weiteres N-, S- oder O-Atom enthalten und substituiert sein kann oder einen ungesättigten 5-gliedrigen Heterocyclus bilden, der 1-3 N-Atome enthalten und substituiert sein kann,
sowie deren Isomeren oder Salze, wobei, falls R⁵-R¹⁰ Wasserstoff ist, R¹ oder R⁴ nicht Methanphosphonsäure oder Ethan-1-phosphonsäure bedeuten.

Die Verbindungen der allgemeinen Formel I beinhalten auch die möglichen tautomeren Formen und umfassen die E- oder Z-Isomeren oder, falls ein chirales Zentrum vorhanden ist, die Razemate oder Enantiomeren.

Die Substituenten stehen bevorzugt in 6- und/oder 7-Stellung.

Der Substituent R² steht ein- bis zweifach, gleich oder verschieden in beliebiger Position am Alkyl-, Alkenyl-, Alkinyl-, Cycloalkyl-, Hetaryl-, Aryl- oder (CH₂)ₙ-Rest.

Unter Alkyl ist jeweils ein geradkettiger oder verzweigter Alkylrest zu verstehen wie beispielsweise Methyl, Ethyl, Propyl, Isopropyl, Butyl, Isobutyl, sek. Butyl, Pentyl, Isopentyl, Hexyl, Heptyl, Octyl, Nonyl, Decyl, wobei C₁₋₆-Alkylreste bevorzugt werden.

Alkenyl beinhaltet insbesondere C₂₋₆-Alkenylreste, die geradkettig oder verzweigt sein können wie beispielsweise 2-Propenyl, 2-Butenyl, 3-Methyl-2-propenyl, 1-Propenyl, 1-Butenyl, Vinyl.

Als Alkinylreste sind insbesondere Ethinyl, 1-Propinyl, 2-Propinyl, 1-Butinyl mit 2-4 Kohlenstoffatomen geeignet.

Unter C₃₋₇-Cycloalkyl ist jeweils Cyclopropyl, Cyclobutyl, Cyclopentyl, Cyclohexyl und Cycloheptyl gemeint, insbesondere C₃₋₅-Cycloalkyl.

Als Arylrest seien beispielsweise Phenyl, Naphthyl, Biphenyl und Indenyl genannt, insbesondere (CH₂)ₙ-Phenyl mit n = 0, 1 oder 2.

Als Hetarylrest sind 5- oder 6-gliedrige Heteroaromaten mit 1-3 Stickstoffatomen geeignet wie beispielsweise Pyrazol, Imidazol, Pyrazin, Pyridin, Pyrimidin, Pyridazin, Triazin.

Unter Halogen ist jeweils Fluor, Chlor, Brom und Jod, insbesondere Fluor, Chlor und Brom, zu verstehen.

Bilden R¹⁴, R¹⁵ und R¹⁶, R¹⁷ gemeinsam mit dem Stickstoffatom einen gesättigten Heterocyclus, so ist beispielsweise Piperidin, Pyrrolidin, Morpholin, Thiomorpholin, Hexahydroazepin oder Piperazin gemeint. Als Substituenten des Heterocyclus seien C₁₋₄-Alkylgruppen genannt wie N-Methyl-piperazin, 2,6-Dimethylmorpholin, oder Arylgruppen wie Phenylpiperazin. Der Substituent kann ein- bis dreifach stehen.

Bilden R¹⁴, R¹⁵ und R¹⁶, R¹⁷ gemeinsam mit dem Stickstoffatom einen ungesättigten Heterocyclus, so seien beispielsweise Imidazol, Pyrazol, Pyrrol und Triazol genannt, die ein-bis zweifach mit Cyano, C₁₋₄-Alkyl, Phenyl oder CO₂C₁₋₆-Alkyl substituiert sein können.

Bevorzugt sind Verbindungen mit R² in der Bedeutung -COR³ oder -POXY und R¹/R⁴ in der Bedeutung von Alkyl.

Als Salze sind die physiologisch verträglichen Salze organischer und anorganischer Basen geeignet wie beispielsweise die gut löslichen Alkali- und Erdalkalisalze sowie N-Methylglukamin, Dimethyl-glukamin, Ethyl-glukamin, Lysin, 1,6-Hexadiamin, Ethanolamin, Glukosamin, Sarkosin, Serinol, Tris-hydroxy-methyl-amino-methan, Aminopropandiol, Sovak-Base, 1-Amino-2,3,4-butantriol.

Ist eine basische Funktion enthalten, sind die physiologisch verträglichen Salze organischer und anorganischer Säuren geeignet wie HCl, H₂SO₄, Phosphorsäure, Zitronensäure, Weinsäure u.a.

Die Verbindungen der Formel I sowie deren physiologisch verträglichen Salze sind auf Grund ihrer Affinität zu den AMPA- oder Kainat-Rezeptoren als Arzneimittel verwendbar.

Auf Grund ihres Wirkprofils eignen sich die erfindungsgemäßen Verbindungen zur Behandlung von Krankheiten, die durch Hyperaktivität excitatorischer Aminosäuren wie zum Beispiel Glutamat oder Aspartat hervorgerufen werden. Da die neuen Verbindungen als Antagonisten excitatorischer Aminosäuren wirken und eine hohe spezifische Affinität zu den AMPA-Rezeptoren zeigen, indem sie den radioaktiv markierten spezifischen Agonisten (RS)α-Amino-3-hydroxy-5-methyl-4-isoxazolpropionat (AMPA) von den AMPA-Rezeptoren verdrängen, eignen sie sich insbesondere zur Behandlung von solchen Krankheiten, die über die Rezeptoren excitatorischer Aminosäuren, insbesondere den AMPA-Rezeptor, beeinflußt werden wie zur Behandlung neurologischer und psychiatrischer Erkrankungen. Zu den neurologischen Erkrankungen , die funktionell und präventiv behandelt werden können, gehören zum Beispiel neurodegenerative Störungen wie Morbus Parkinson, Morbus Alzheimer, Chorea Huntington, Amyotrophe Lateralsklerose und Olivopontocerebelläre Degeneration. Erfindungsgemäss können die Verbindungen zur Prävention des postischämischen Zelluntergangs, des Zelluntergangs nach Hirntrauma, bei Schlaganfall, Hypoxie, Anoxie und Hypoglykämie und zur Behandlung der Senilen Demenz, Multiinfarkt Demenz sowie Epilepsie und Muskelspastik verwendet werden. Zu den psychiatrischen Erkrankungen gehören Angstzustände, Schizophrenie, Migräne, Schmerzzustände sowie die Behandlung von Schlafstörungen oder der Entzugssymptomatik nach Drogenmißbrauch wie bei Alkohol-, Kokain-, Benzodiazepin- oder Opiat-Entzug.

Zur Verwendung der erfindungsgemäßen Verbindungen als Arzneimittel werden diese in die Form eines pharmazeutischen Präparats gebracht, das neben dem Wirkstoff für die enterale oder parenterale Applikation geeignete pharmazeutische, organische oder anorganische inerte Trägermaterialien, wie zum Beispiel, Wasser, Gelantine, Gummi arabicum, Milchzucker, Stärke, Magnesiumstearat, Talk, pflanzliche Öle, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie drüber hinaus Hilfsstoffe wie Konservierungs-, Stabilisierungs-, Netzmittel oder Emulgatoren, Salze zur Veränderung des osmotischen Drucks oder Puffer.

Für die parenterale Anwendung sind insbesondere Injektionslösungen oder Suspensionen, insbesondere wäßrige Lösungen der aktiven Verbindungen in polyhydroxyethoxyliertem Rizinusöl, geeignet.

Als Trägersysteme können auch grenzflächenaktive Hilfsstoffe wie Salze der Gallensäuren oder tierische oder pflanzliche Phospholipide, aber auch Mischungen davon sowie Liposome oder deren Bestandteile verwendet werden.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt ist.

Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 0,5-1000 mg, vorzugsweise 50-200 mg, wobei die Dosis als einmal zu verabreichende Einzeldosis oder unterteilt in 2 oder mehreren Tagesdosen gegeben werden kann.

Die Herstellung der erfindungsgemäßen Verbindung erfolgt nach an sich bekannten Methoden. Beispielsweise gelangt man zu Verbindungen der Formel I dadurch, daß man
a) eine Verbindung der Formel II oder III 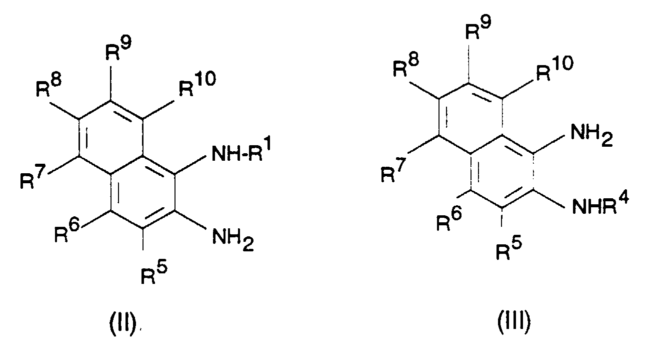 worin R¹ bis R¹⁰ die obige Bedeutung haben mit Oxalsäure oder reaktiven Oxalsäurederivaten cyclisiert oder
b) eine Verbindung der Formel IV oder V 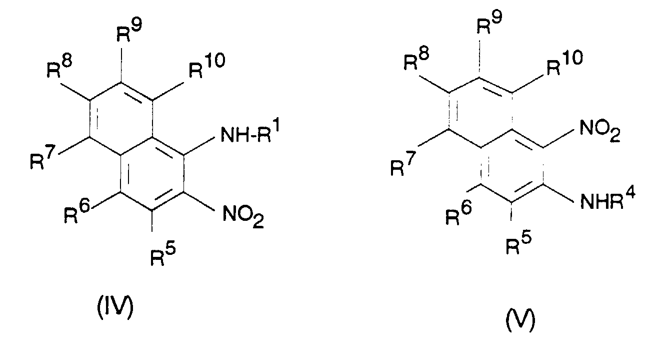 worin R¹ bis R¹⁰ die obige Bedeutung haben mit Oxalsäure oder reaktiven Oxalsäurederivaten umsetzt und nach Reduktion der Nitrogruppe cyclisiert oder
c) eine Verbindung der Formel VI 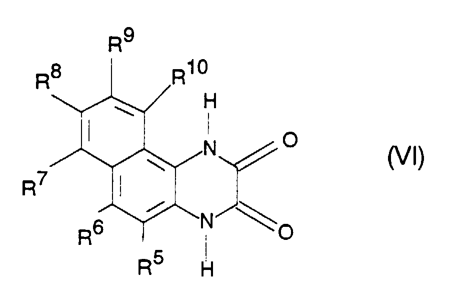 worin R⁵ bis R¹⁰ die obige Bedeutung haben in Gegenwart einer Base mit R¹ Z oder R⁴ Z, wobei Z eine Fluchtgruppe darstellt, umsetzt und gewünschtenfalls anschließend die Estergruppe verseift oder die Säuregruppe verestert oder amidiert oder die Nitrogruppe reduziert zur Aminogruppe oder die Aminogruppe alkyliert oder acyliert oder die Aminogruppe gegen Halogen oder Cyano austauscht oder eine Nitrogruppe oder Halogen einführt oder ein Nitril in das Tetrazol oder Amidoxim überführt oder nucleophil substituiert oder die Isomeren trennt oder die Salze bildet.

Die Cyclisierung der Verbindungen der Formel II und III mit Oxalsäure oder einem reaktiven Oxalsäurederivat erfolgt einstufig oder auch zweistufig. Als bevorzugt ist das Zweistufenverfahren zu betrachten, bei dem das Diamin mit einem Oxalsäurederivat wie dem Oxalesterhalbchlorid oder reaktiven Oxalsäureimidazolidderivaten in polaren Lösungsmitteln wie cyclischen oder acyclischen Ethern oder halogenierten Kohlenwasserstoffen beispielsweise Tetrahydrofuran, Diethylether oder Methylenchlorid in Gegenwart einer Base wie organischen Aminen beispielsweise Triethylamin, Pyridin, Hünig-Base oder Diethylaminopyridin umgesetzt wird. Die anschließende Cyclisierung kann basisch oder auch sauer, vorzugsweise aber in saurem Milieu durchgeführt werden, wobei dem Lösungsmittel Alkohol zugesetzt werden kann.

Geeignete Basen für das Zweistufenverfahren stellen auch Alkalihydride dar wie NaH, die in inerten Lösungsmitteln beispielsweise Kohlenwasserstoffen und Ethern wie Dimethoxyethan, THF u.a. eingesetzt werden.

In der Verfahrensvariante b) wird nach der Acylierung mit Oxalsäure oder dem reaktiven Oxalsäurederivat in üblicher Weise die Nitrogruppe katalytisch oder durch Reduktion mit Eisenpulver in Essigsäure bei erhöhter Temperatur oder auch mit Natriumsulfid und Ammoniumhydroxid in Alkohol reduziert und wie oben beschrieben cyclisiert.

Die Einführung der Substituenten R¹ und R⁴ erfolgt gemäß dem Verfahren c) nach den üblichen Alkylierungsmethoden, indem man das Chinoxalindion mit R¹- oder R⁴-Z, worin Z beispielsweise Tosylat, Mesylat, Triflat, Nonaflat oder Halogen bedeutet, in Gegenwart von Basen bei Raumtemperatur oder erhöhter Temperatur in aprotischen Lösungsmitteln umsetzt. Das Anion kann auch erzeugt werden, bevor R¹- oder R⁴-Z zugegeben werden. Als Basen sind beispielsweise Alkaliverbindungen wie Kaliumcarbonat, Natriumhydroxid, Alkalialkoholate und insbesondere Metallhydride wie Natriumhydrid geeignet. Eventuell können die Alkaliverbindungen auch unter Phasentransferbedingungen umgesetzt werden. Erhält man Gemische von Verbindungen mit dem Substituenten R¹ bzw. R⁴, so werden diese in üblicher Weise getrennt. Für die Umsetzung geeignete Lösungsmittel sind aprotische polare Lösungsmittel wie Dimethylformamid, N-Methylpyrrolidon aber auch cyclische Ether wie Dioxan oder Tetrahydrofuran.

Wird in der Verfahrensvariante c) mit 2 Mol R¹-Z unter sonst analogen Reaktionsbedingungen umgesetzt, so werden gleichzeitig die Substituenten R¹ und R⁴ eingeführt.

Die sich gegebenenfalls anschließende Verseifung einer Estergruppe kann basisch oder vorzugsweise sauer erfolgen, indem man bei erhöhter Temperatur bis zur Siedetemperatur des Reaktionsgemisches in Gegenwart von Säuren wie hoher konzentrierter wäßriger Salzsäure in Lösungsmitteln wie beispielsweise Trifluoressigsäure oder Alkoholen hydrolysiert. Phosphonsäureester werden bevorzugt durch Erhitzen in hochkonzentrierten wäßrigen Säuren wie zum Beispiel konzentrierter Salzsäure oder durch Behandlung mit trimethylsilylhalogenid und anschließende Behandlung mit Wasser hydrolysiert.

Die Veresterung der Carbonsäure oder Phosphonsäure geschieht in an sich bekannter Weise mit dem entsprechenden Alkohol in Säure oder in Gegenwart eines aktivierten Säuredrivats. Als aktivierte Säurederivate kommen zum Beispiel Säurechlorid, -imidazolid oder -anhydrid in Frage. Bei den Phosphonsäuren kann man die Veresterung durch Umsetzung mit Orthoestern gegebenenfalls unter Zusatz von Katalysatoren wie p-Toluolsulfonsäuree erreichen.

Die Amidierung erfolgt an den freien Säuren oder an deren reaktiven Derivaten wie beispielsweise Säurechloriden, gemischten Anhydriden, Imidazoliden oder Aziden durch Umsetzung mit den entsprechenden Aminen bei Raumtemperatur.

Die Reduktion der Nitrogruppe zur Aminogruppe erfolgt katalytisch in polaren Lösungsmitteln bei Raumtemperatur oder erhöhter Temperatur unter Wasserstoffdruck. Als Katalysatoren sind Metalle wie Raney-Nickel oder Edelmetallkatalysatoren wie Palladium oder Platin gegebenenfalls in Gegenwart von Bariumsulfat oder auf Trägern geeignet. Statt Wasserstoff kann auch Ammoniumformiat in bekannter Weise benutzt werden. Reduktionsmittel wie Zinn-II-chlorid oder Titan-III-chlorid können ebenso verwendet werden wie komplexe Metallhydride eventuell in Gegenwart von Schwermetallsalzen. Es kann vorteilhaft sein vor der Reduktion die Estergruppe einzuführen. Nitrogruppen können auch selektiv in üblicher Weise mit Na₂S oder Natriumdithionit reduziert werden.

Wird eine Alkylierung einer Aminogruppe gewünscht, so kann nach üblichen Methoden beispielsweise mit Alkylhalogeniden oder nach der Mitsonubo Variante durch Umsetzung mit einem Alkohol in Gegenwart von Tiphenylphosphin und Azodicarbonsäureester alkyliert werden oder man unterwirft das Amin einer reduktiven Aminierung mit Aldehyden oder Ketonen gegebenenfalls nacheinander mit zwei verschiedenen Carbonylverbindungen, wobei man gemischte Derivate erhält (Literatur z.B. Verardo et al. Synthesis 1993, 121; Synthesis 1991, 447; Kawaguchi, Synthesis 1985, 701; Micovic et al. Synthesis 1991, 1043).

Die Acylierung einer Aminogruppe erfolgt in üblicher Weise beispielsweise mit einem Säurehalogenid oder Säureanhydrid gegebenenfalls in Gegenwart einer Base wie Dimethylaminopyridin in Lösungsmitteln wie Methylenchlorid, Tetrahydrofuran oder Pyridin oder nach der Scotten Baumann Reaktion.

Die Einführung der Cyanogruppe kann mit Hilfe der Sandmeyer-Reaktion erfolgen; beipsielsweise kann man die aus den Aminoverbindungen mit Nitriten intermediär gebildeten Diazoniumsalze mit Alkalicyaniden in Gegenwart von Cu-I-cyanid umsetzen.

Die Einführung der Halogene Chlor, Brom oder Jod über die Aminogruppe kann beispielsweise auch nach Sandmeyer erfolgen, indem man die mit Nitriten intermediär gebildete Diazoniumsalze mit Cu(I)chlorid oder Cu(I)bromid in Gegenwart der entsprechenden Säure wie Salzsäure oder Bromwasserstoffsäure umsetzt oder mit Kaliumjodid umsetzt.

Wenn ein organischer Salpetrigsäureester benutz wird, kann man die Halogen z.B. durch Zusatz von Methylenjodid oder Tetrabrommethan einführen in einem Lösungsmittel wie zum Beispiel Dimethylformamid.

Die Einführung von Fluor gelingt beispielsweise durch Balz-Schiemann-Reaktion des Diazoniumtetrafluorborates.

Die Einführung einer NO₂-Gruppe gelingt durch eine Reihe von bekannten Nitrierungsmethoden. Beispielsweise kann mit Nitroniumtetrafluoroborat in inerten Lösungsmitteln wie halogenierten Kohlenwasserstoffen oder in Sulfolan, Eisessig oder Acetonitril nitriert werden. Möglich ist auch die Einführung z.B.durch Nitriersäure in Wasser oder konz. Schwefelsäure als Lösungsmittel bei Temperaturen zwischen 0°C und 30°C.

Die Einführung von Halogen gelingt durch bekannte Halogenierungsmethoden wie z.B. durch elektrophile aromatische Substitution.
Beispielsweise kann nach einem Verfahren mit Jod und Jodsäure von Wirth et al. [Liebigs Ann. Chem. 634, 84 (1960)] oder mit N-Jodsuccinimid in Lösungsmitteln wie Tetrahydrofuran, Dimethylformamid oder Trifluormethansulfonsäure jodiert werden.

Die Einführung des Tetrazoles gelingt durch Umsetzung des entsprechenden Nitriles mit einem Azid wie z.B. Trimethylsilylazid, Stickstoffwasserstoffsäure oder Natriumazid gegebenenfalls unter Zusatz einer Protonenquelle wie z.B. Ammoniumchlorid oder Triethylammoniumchlorid in polaren Lösungsmitteln wie Dimethylformamid, Dimethylacetamid oder N-Methylpyrrolidon bei Temperaturen bis zum Siedepunkt des Lösungsmittels.

Amidoxime stellt man aus dem entsprechenden Nitril mit Hydroxylaminhydrochlorid z.Bsp. in Alkohol-Wasser-Mischungen als Lösungsgmittel dar.

Die nucleophile Substitution wird nach literaturbekannten Methoden in Gegenwart einer Base durchgeführt und wird duch eine aktivierende elektronenziehende Gruppe wie z.B. Nitro, Cyano, Trifluormethyl vorzugsweise in o-Stellung begünstigt.Als Nucleophile sind beispielsweise primäre und sekundäre Amine, N-enthaltende ungesättigte und gesättigte Heterocyclen, Cyanid, Alkoholate, Thiolate u.a. geeignet, Die Umsetzung kann in polaren Lösungsmitteln wie Alkoholen, halogenierten Kohlenwasserstoffen, Dimethylacetamid, Acetonitril oder Wasser oder ohne Lösungsmittel vorgenommen werden. Als Basen sind anorganische Basen wie Alkali- oder Erdalkalihydroxide oder -carbonate oder organische Basen wie cyclische, alicyclische und aromatische Amine, wie DBU, Hünigbase, Pyridin oder Dimethylaminopyridin geeignet. Als Base kann im Fall von Aminen das Nucleophil selbst im Überschuß benutzt werden, wobei man gegebenenfalls ohne weiteres Lösungsmittel arbeiten kann oder unter Druck. Beispielsweise kann das aktivierte Sulfonsäurederivat wie das Sulfonsäurechlorid in üblicher Weise mit nucleophilen N-Derivaten (wie H₂N(C₁₋₄-Alkyl) oder H₂N-CH₂CONH₂ oder H₂N-CH₂-R²) oder mit nucleophilen C-Derivaten (wie CF₃-Anion oder CH₂-CONH₂-Anion) umgesetzt werden.

Die Isomerengemische können nach üblichen Methoden wie beispielsweise Kristallisation, Chromatographie oder Salzbildung in die Enantiomeren bzw. E/Z-Isomeren aufgetrennt werden.

Die Herstellung der Salze erfolgt in üblicher Weise, indem man eine Lösung der Verbindung der Formel I mit der äquivalenten Menge oder einem Überschuß einer Alkali- oder Erdalkali-Verbindung, die gegebenenfalls in Lösung ist, versetzt und den Niederschlag abtrennt oder in üblicher Weise die Lösung aufarbeitet.

Soweit die Herstellung der Ausgangsverbindungen nicht beschrieben wird, sind diese bekannt oder analog zu bekannten Verbindungen wie beispielsweise nach WO 93/08171 oder hier beschriebenen Verfahren herstellbar.

Die nachfolgenden Verfahren sollen die Herstellung der erfindungsgemäßen Verbindungen beispielhaft erläutern:

### Beispiel 1:

### (6-Nitro-2,3-dioxo-1,2,3,4-tetrahydrobenzo[f]chinoxalin-1-yl)-methanphosphonsäure

Die Synthese von 2,4-Dinitro-1-fluornaphthalin erfolgt wie in der Literatur beschrieben: J.Chem. Soc. 1962, 2616
Die Synthese von 2,4-Dinitro-1-chlornaphthalin erfolgt wie in der Literatur beschrieben: Berichte 1908, 41, 3932
Die Synthese von Toluolsulfonsäure-(2,4-dinitronaphth-1-ol)-ester erfolgt wie in der Literatur beschrieben: Berichte 1908, 41, 3932

### a) Trifluormethansulfonsäure-(2,4-dinitronaphth-1-ol)-ester

2 g 2,4-Dinitronaphth-1-ol (Martius-Gelb) werden in 30 ml Dichlormethan mit 1,72 ml = 1,2 Equivalenten Trifluormethansulfonsäureanhydrid und 1,43 ml Triethylamin versetzt und bei Raumtemperatur gerührt bis zum Verschwinden des Edukts. Es wird mit Dichlormethan verdünnt, neutralisiert, mit Sole gewaschen und das Lösemittel abgezogen. Das Rohprodukt wird aus heißem Ethanol umkristallisiert. Man erhält 89% Produkt.
Auf analoge Weise entsteht aus Nonafluorbutansulfonylfluorid in Toluol :
Nonafluorbutansulfonsäure-(2,4-dinitronaphth-1-ol)-ester

### b) (2,4 Dinitro-1-naphthylamino)-methanphosphonsäurediethylester

4 g 2,4-Dinitro-1-chlornaphthalin ( 15,84 mMol) werden in 6,5 g Aminomethanphosphonsäurediethylester und 4 g Diphenylmethan gelöst und 72 Stunden bei Raumtemperatur gerührt. Der Ansatz wird mit Methylenchlorid verdünnt, mit 1-n NaOH und zweimal mit Sole gewaschen, getrocknet und einrotiert. Das Rohprodukt wird mit 3,5 Litern Cyclohexan und Essigester 1:1 als Laufmittel über Kieselgel chromatographiert. Man erhält in mehreren Fraktionen 68% Produkt als zähes Öl. NMR (DMSO,Delta in ppm):1.15(6H,tr),4(4H dq)4.25 (2H m), 7.8, 7.98, 8.9, 9.05 je 1 H, 8.6 (2H,d)
In gleicher Art und Weise werden hergestellt:
1-Phenyl-1-(2,4 dinitro-1-naphthylamino)-methanphosphonsäurediethylester
1-(2,4 Dinitro-1-naphthylamino)-ethan-1-phosphonsäurediethylester
1-(2,4 Dinitro-1-naphthylamino)-propan-1-phosphonsäurediethylester
1-(2,4 Dinitro-1-naphthylamino)-butan-1-phosphonsäurediethylester
1-(2,4 Dinitro-1-naphthylamino)-hexan-1-phosphonsäurediethylester

### c) (2-Amino-4-nitro-1-naphthylamino)-methanphosphonsäurediethylester

1,352 g (2,4 Dinitro-1-naphthylamino)-methanphosphonsäurediethylester werden in 32 ml Ethanol und 23 ml Wasser gelöst, dazu fügt man 4,4 ml 25%ige Ammoniumhydroxidlösung, 1,61 g Ammoniumchlorid und 2,81 g 35%iges Natriumsulfid und erwärmt die Lösung einige Stunden auf 90°. Das Ethanol-Wassergemisch wird am Rotationsverdampfer eingeengt, in Wasser aufgenommen, mit Essigester extrahiert, mit Sole gewaschen, getrocknet und einrotiert.Das Rohprodukt wird mit 2 Litern Cyclohexan und Essigester 1:1 und 1 Liter reinem Essigester als Laufmittel über Kieselgel chromatographiert. Man erhält in mehreren Fraktionen 262 mg Produkt als braunen Feststoff.
In gleicher Art und Weise werden hergestellt:
1-(2-Amino-4-nitro-1-naphthylamino)-ethan-1-phosphonsäurediethylester
1-(2-Amino-4-nitro-1-naphthylamino)-butan-1-phosphonsäurediethylester
1-(2-Amino-4-nitro-1-naphthylamino)-hexan-1-phosphonsäurediethylester
1-Phenyl-1-(2-amino-4-nitro-1-naphthylamino)-methanphosphonsäurediethylester
1-(2-Amino-4-nitro-1-naphthylamino)-propan-1-phosphonsäurediethylester (79% Ausbeute)

### d) (6-Nitro-2,3-dioxo-1,2,3,4-tetrahydrobenzo[f]chinoxalin-1-yl)-methanphosphonsäurediethylester

100 mg (2-Amino-4-nitro-1-naphthylamino)-methanphosphonsäurediethylester werden in wenigen ml Tetrahydrofuran gelöst und bei Eisbadtemperatur mit 95 Mikrolitern destilliertem Ethyloxalychlorid und 118 Mikrolitern Triethylamin behandelt. Man rührt die Lösung zunächst auf Raumtemperatur und läßt dann bei 55° Ölbadtemperatur die Umsetzung vervollständigen. Der Ansatz wird eingeengt, in Essigester aufgenommen und mit Sole gewaschen, getrocknet und einrotiert.Das Rohprodukt wird mit Ethanol als Laufmittel über Kieselgel chromatographiert. Man erhält als polare Fraktion 76 mg oder 67% d.Theorie an Produkt. NMR (DMSO,Delta in ppm):1.03 (6H tr), 3.8 (4H dq), 4.93 (2H d),7.73 (2H dd), 8.2, 8.35, 8.47 je 1H.
In gleicher Art und Weise werden hergestellt:
1-(6-Nitro-2,3-dioxo-1,2,3,4-tetrahydrobenzo[f]chinoxalin-1-yl)-ethan-1-phosphonsäurediethylester vom Schmelzpunkt > 300° (Zersetzung)
1-(6-Nitro-2,3-dioxo-1,2,3,4-tetrahydrobenzo[f]chinoxalin-1-yl)-propan-1-phosphonsäurediethylester vom Schmelzpunkt >300° (Zers)
1-(6-Nitro-2,3-dioxo-1,2,3,4-tetrahydrobenzo[f]chinoxalin-1-yl)-butan-1-phosphonsäurediethylester
1-(6-Nitro-2,3-dioxo-1,2,3,4-tetrahydrobenzo[f]chinoxalin-1-yl)-hexan-1-phosphonsäurediethylester
1-Phenyl-1-(6-Nitro-2,3-dioxo-1,2,3,4-tetrahydrobenzo[f]chinoxalin-1-yl)-methanphosphonsäurediethylester

### e) (6-Nitro-2,3-dioxo-1,2,3,4-tetrahydrobenzo[f]chinoxalin-1-yl)-methanphosphonsäure

32 mg (6-Nitro-2,3-dioxo-1,2,3,4-tetrahydrobenzo[f]chinoxalin-1-yl)-methanphosphonsäurediethylester werden in 2 ml Acetonitril unter Stickstoff bei Raumtemperatur vorgelegt. 80 Mikroliter Trimethylsilylbromid werden durch ein Septum zugetropft. Die Lösung wird 20 Stunden bei Raumtemperatur gerührt und eingeengt. Der Rückstand wird in wenig Wasser suspendiert, abgesaugt und mit Wasser gewaschen. Nach Trocknen des Rohproduktes erhält man 21 mg Phosphonsäure.NMR (DMSO,Delta in ppm): 4.7 (2H d),7.65 (2H m), 8.2 (1H,m) 8.44 (2H).Fp. >340°
In entsprechender Art und Weise werden hergestellt:
1-(6-Nitro-2,3-dioxo-1,2,3,4-tetrahydrobenzo[f]chinoxalin-1-yl)-ethan-1-phosphonsäure mit Schmelzpunkt >300°C
1-(6-Nitro-2,3-dioxo-1,2,3,4-tetrahydrobenzo[f]chinoxalin-1-yl)-propan-1-phosphonsäure mit Schmelzpunkt >300°C
1-(6-Nitro-2,3-dioxo-1,2,3,4-tetrahydrobenzo[f]chinoxalin-1-yl)-butan-1-phosphonsäure
1-(6-Nitro-2,3-dioxo-1,2,3,4-tetrahydrobenzo[f]chinoxalin-1-yl)-hexan-1-phosphonsäure
1-Phenyl-1-(6-Nitro-2,3-dioxo-1,2,3,4-tetrahydrobenzo[f]chinoxalin-1-yl)-methanphosphonsäure

### Beispiel 2:

### 1-(2,4 Dinitro-1-naphth-yl-[N-ethoxyoxalyl]amino)-propan-1-phosphonsäurediethylester

Zu 100 mg (o,24 mMol) 1-(2,4 Dinitro-1-naphthylamino)-propan-1-phosphonsäurediethylester in 1,5 ml Tetrahydrofuran gibt man nach und nach insgesamt 1,2 mMol Triethylamin sowie 1,2 mMol Ethyloxalylchlorid und rührt insgesamt 20 Stunden bei 80° Ölbadtemperatur. Das Lösemittel wird eingeengt, das Rohprodukt zwischen Wasser und viel Essigester verteilt, die organische Phase mit Sole gewaschen, getrocknet und einrotiert. Das entstandene Gemisch wird mit Essigester als Laufmittel über Kieselgel chromatographiert. Man erhält als polare Fraktion 24 mg an Produkt sowie etwas Edukt.
In analoger Weise entsteht 2,4 Dinitro-1-naphth-yl-[N-ethoxyoxalyl]aminomethanphosphonsäurediethylester

### Beispiel 3:

### (6,10-Dinitro-2,3-dioxo-1,2,3,4-tetrahydrobenzo[f]chinoxalin-1-yl)-methanphosphonsäure

### a) (6,10-Dinitro-2,3-dioxo-1,2,3,4-tetrahydrobenzo[f]chinoxalin-1-yl)-methanphosphonsäurediethylester

90 mg (6-Nitro-2,3-dioxo-1,2,3,4-tetrahydrobenzo[f]chinoxalin-1-yl)-methanphosphonsäurediethylester werden in 1 ml Methylenchlorid gelöst und mit 30 mg Nitroniumtetrafluorborat bei Eisbadtemperatur und später bei Raumtemperatur gerührt.
Mit Natriumhydrogencarbonatlösung wird auf pH 8 eingestellt ,die organische Phase mit Sole gewaschen, getrocknet und einrotiert. Das entstandene Gemisch wird über Kieselgel chromatographiert. Man erhält 24 mg Produkt
In analoger Weise entsteht 1-(6,10-Dinitro-2,3-dioxo-1,2,3,4-tetrahydrobenzo[f]chinoxalin-1-yl)-ethan-1-phosphonsäurediethylester

### b) In analoger Durchführung wie unter Beispiel 1 e) beschrieben werden die Ester verseift. Man erhält

(6,10-Dinitro-2,3-dioxo-1,2,3,4-tetrahydrobenzo[f]chinoxalin-1-yl)-methanphosphonsäure und analog
1-(6,10-Dinitro-2,3-dioxo-1,2,3,4-tetrahydrobenzo[f]chinoxalin-1-yl)-ethan-1-phosphonsäure

### Beispiel 4:

### (6-Nitro-7-sulfamoyl-2,3-dioxo-1,2,3,4-tetrahydrobenzo[f]chinoxalin-1-yl)-methanphosphonsäure und (7-Sulfamoyl-2,3-dioxo-1,2,3,4-tetrahydrobenzo[f]chinoxalin-1-yl)-methanphosphonsäure

### a) 2-Nitro-5-sulfonamido-naphth-1-ol und 2,4-Dinitro-5-sulfonamido-naphth-1-ol

1 g 5-Sulfonamidonaphth-1-ol wird mit einer Mischung von Salpetersäure und Wasser unter Eisbadkühlung nitriert. Die Mischung wird mit festem Natriumhydrogencarbonat neutralisiert, mit viel Methylenchlorid extrahiert,mit Sole gewaschen, getrocknet und einrotiert. Das entstandene Gemisch wird über Kieselgel chromatographiert. Es resultiert eine Mischung von 2-Nitro-5-sulfonamido-naphth-1-ol und 2,4-Dinitro-5-sulfonamido-naphth-1-ol. NMR (DMSO,Delta in ppm): 7.8 (4H m),8.2 (2H m), 8.4 (1H,d) 8.7 (1H d) bzw. 7.05 (2H s) 7.6 (1H dd),8.3 (1H d), 8.5 (1H,d) 8.8 (1H s) für das Dinitronaphthol.

### b) In analoger Durchführung wie in der Literatur beschrieben werden die Toluolsulfonsäureester dargestellt. Man erhält

Toluolsulfonsäure-(2,4-dinitro-5-sulfonamido-naphth-1-ol)-ester sowie
Toluolsulfonsäure-(2-nitro-5-sulfonamido-naphth-1-ol)-ester

### c) In analoger Durchführung wie unter Beispiel 1 b) beschrieben werden die Ester mit Aminoalkanphosphonsäurediethylestern umgesetzt. So entstehen

1-Phenyl-1-(2,4 dinitro-5-sulfamoyl-1-naphthylamino)-methanphosphonsäurediethylester
1-Methyl-1-(2,4 dinitro-5-sulfamoyl-1-naphthylamino)-ethan-1-phosphonsäurediethylester (2,4 Dinitro-5-sulfamoyl-1-naphthylamino)-methanphosphonsäurediethylester
1-(2,4 Dinitro-5-sulfamoyl-1-naphthylamino)-propan-1-phosphonsäurediethylester

### beziehungsweise

1-Phenyl-1-(2-nitro-5-sulfamoyl-1-naphthylamino)-methanphosphonsäurediethylester
1-Methyl-1-(2-nitro-5-sulfamoyl-1-naphthylamino)-ethan-1-phosphonsäurediethylester (2-Nitro-5-sulfamoyl-1-naphthylamino)-methanphosphonsäurediethylester
1-(2-Nitro-5-sulfamoyl-1-naphthylamino)-propan-1-phosphonsäurediethylester

### d) Mit gleichen Methoden wie unter Beispiel 1 c) beschrieben erhält man

1-Phenyl-1-(2 amino-4-nitro-5-sulfamoyl-1-naphthylamino)-methanphosphonsäurediethylester
1-Methyl-1-(2 amino-4-nitro-5-sulfamoyl-1-naphthylamino)-ethan-1-phosphonsäurediethylester
(2-Amino-4-nitro-5-sulfamoyl-1-naphthylamino)-methanphosphonsäurediethylester
1-(2-Amino-4-nitro-5-sulfamoyl-1-naphthylamino)-propan-1-phosphonsäurediethylester

### beziehungsweise

1-Phenyl-1-(2-amino-5-sulfamoyl-1-naphthylamino)-methanphosphonsäurediethylester
1-Methyl-1-(2-amino-5-sulfamoyl-1-naphthylamino)-ethan-1-phosphonsäurediethylester (2-Amino-5-sulfamoyl-1-naphthylamino)-methanphosphonsäurediethylester
1-(2-Amino-5-sulfamoyl-1-naphthylamino)-propan-1-phosphonsäurediethylester

### e) Durch Umsetzung mit reaktiven Oxalsäurederivaten wie beispielsweise in Beispiel 1 d) beschrieben oder nach bekannten Verfahren entstehen

1-(6-Nitro-7-sulfamoyl-2,3-dioxo-1,2,3,4-tetrahydrobenzo[f]chinoxalin-1-yl)ethan-1-phosphonsäurediethylester
1-(6-Nitro-7-sulfamoyl-2,3-dioxo-1,2,3,4-tetrahydrobenzo[f]chinoxalin-1-yl)-propan-1-phosphonsäurediethylester
1-Phenyl-1-(6-nitro-7-sulfamoyl-2,3-dioxo-1,2,3,4-tetrahydrobenzo[f]chinoxalin-1-yl)-methanphosphonsäurediethylester
(6-Nitro-7-sulfamoyl-2,3-dioxo-1,2,3,4-tetrahydrobenzo[f]chinoxalin-1-yl)-methanphosphonsäurediethylester
sowie
1-(7-Sulfamoyl-2,3-dioxo-1,2,3,4-tetrahydrobenzo[f]chinoxalin-1-yl)-ethan-1-phosphonsäurediethylester
1-(7-Sulfamoyl-2,3-dioxo-1,2,3,4-tetrahydrobenzo[f]chinoxalin-1-yl)-propan-1-phosphonsäurediethylester
1-Phenyl-1-(7-sulfamoyl-2,3-dioxo-1,2,3,4-tetrahydrobenzo[f]chinoxalin-1-yl)-methanphosphonsäurediethylester
(7-Sulfamoyl-2,3-dioxo-1,2,3,4-tetrahydrobenzo[f]chinoxalin-1-yl)-methanphosphonsäurediethylester

### f) In analoger Durchführung wie unter Beispiel 1 e) beschrieben werden die Ester verseift. Man erhält

1-(6-Nitro-7-sulfamoyl-2,3-dioxo-1,2,3,4-tetrahydrobenzo[f]chinoxalin-1-yl)-ethan-1-phosphonsäure
1-(6-Nitro-7-sulfamoyl-2,3-dioxo-1,2,3,4-tetrahydrobenzo[f]chinoxalin-1-yl)-propan-1-phosphonsäure
1-Phenyl-1-(6-nitro-7-sulfamoyl-2,3-dioxo-1,2,3,4-tetrahydrobenzo[f]chinoxalin-1-yl)-methanphosphonsäure
(6-Nitro-7-sulfamoyl-2,3-dioxo-1,2,3,4-tetrahydrobenzo[f]chinoxalin-1-yl)-methanphosphonsäure
sowie
1-(7-sulfamoyl-2,3-dioxo-1,2,3,4-tetrahydrobenzo[f]chinoxalin-1-yl)-ethan-1-phosphonsäure
1-(7-sulfamoyl-2,3-dioxo-1,2,3,4-tetrahydrobenzo[f]chinoxalin-1-yl)-propan-1-phosphonsäure
1-Phenyl-1-(7-sulfamoyl-2,3-dioxo-1,2,3,4-tetrahydrobenzo[f]chinoxalin-1-yl)-methanphosphonsäure
(7-sulfamoyl-2,3-dioxo-1,2,3,4-tetrahydrobenzo[f]chinoxalin-1-yl)-methanphosphonsäure

### Beispiel 5:

(6-Nitro-9-sulfamoyl-2,3-dioxo-1,2,3,4-tetrahydrobenzo[f]chinoxalin-1-yl)-methanphosphonsäure aus gekaufter Flaviansäure (5,7-Dinitro-8-hydroxy-naphthalin-2-sulfonsäure) wird nach Schutz der Sulfonsäure in analoger Weise dargestellt.

### Beispiel 6:

1-(6-Nitro-9-sulfamoyl-2,3-dioxo-1,2,3,4-tetrahydrobenzo[f]chinoxalin-1-yl)-ethan-1-phosphonsäure aus gekaufter Flaviansäure (5,7-Dinitro-8-hydroxy-naphthalin-2-sulfonsäure) wird analog Beispiel 5 dargestellt.

### Beispiel 7:

### (6-Amino-2,3-dioxo-1,2,3,4-tetrahydrobenzo[f]chinoxalin-1-yl)-methanphosphonsäure aus (6-Nitro-2,3-dioxo-1,2,3,4-tetrahydrobenzo[f]chinoxalin-1-yl)-methanphosphonsäure

a) Man löst 0,3 mMol ( 6-Nitro-2,3-dioxo-1,2,3,4-tetrahydrobenzo[f]chinoxalin-1-yl)-methanphosphonsäurediethylester in 10 ml DMF z.A. und fügt 10 mg Platinoxidkatalysator hinzu, der mit Wasserstoff vorgesättigt wurde. Es wird drei Stunden bei Normaldruck und RT mit Wasserstoff hydriert. Der Katalysator wird über Celite abgesaugt, die Mutterlauge wird mit Wasser verdünnt und der entstandene Niederschlag abgesaugt. Man erhält 44% der Theorie an (6-Amino-2,3-dioxo-1,2,3,4-tetrahydro-benzo[f]chinoxalin-1-yl)-methanphosphonsäurediethylester. Dieser wird wie in Beispiel 1e) beschrieben oder nach Standardbedingungen mit HCl zur (6-Amino-2,3-dioxo-1,2,3,4-tetrahydrobenzo[f]chinoxalin-1-yl)-methanphosphonsäure (respektive zu deren Hydrochlorid) verseift.

### Analog entsteht (6-Amino-2,3-dioxo-1,2,3,4-tetrahydrobenzo[f]chinoxalin-4-yl)-methanphosphonsäure.

### b) Unter Verwendung von Palladium/Bariumsulfat Katalysator entstehen analog:

1-(6-Amino-2,3-dioxo-1,2,3,4-tetrahydrobenzo[f]chinoxalin-1-yl)-acetonitril aus
1-(6-Nitro-2,3-dioxo-1,2,3,4-tetrahydrobenzo[f]chinoxalin-1-yl)-acetonitril und
1-(6-Amino-2,3-dioxo-1,2,3,4-tetrahydrobenzo[f]chinoxalin-4-yl)-acetonitril aus
1-(6-Nitro-2,3-dioxo-1,2,3,4-tetrahydrobenzo[f]chinoxalin-4-yl)-acetonitril

### Beispiel 8:

(6-Cyano-2,3-dioxo-1,2,3,4-tetrahydrobenzo[f]chinoxalin-1-yl)-methanphosphonsäurediethylester wird aus (6-Amino-2,3-dioxo-1,2,3,4-tetrahydrobenzo[f]chinoxalin-1-yl)-methanphosphonsäureester durch Diazotierung und Sandmeyer-Reaktion mit Kupfer(I)cyanid erhalten. Nach Verseifung des Esters mit bekannten Methoden erhält man (6-Cyano-2,3-dioxo-1,2,3,4-tetrahydrobenzo-[f]chinoxalin-1-yl)-methanphosphonsäure.

Analog erhält man (6-Cyano-2,3-dioxo-1,2,3,4-tetrahydrobenzo[f]chinoxalin-4-yl)-methanphosphonsäure aus (6-Cyano-2,3-dioxo-1,2,3,4-tetrahydrobenzo[f]chinoxalin-4-yl)-methanphosphonsäurediethylester.

### Beispiel 9:

### (6-Nitro-7-sulfonamido-2,3-dioxo-1,2,3,4-tetrahydrobenzo[f]chinoxalin-1-yl)-methanphosphonsäurediethylester und (6-Nitro-7-sulfonamido-2,3-dioxo-1,2,3,4-tetrahydrobenzo[f]chinoxalin-4-yl)-methanphosphonsäurediethylester

Man löst 150 mg 6-Nitro-7-sulfonamido-2,3-dioxo-1,2,3,4-tetrahydrobenzo[f]chinoxalin, das nach bekannten Methoden synthetisierbar ist, in 1 ml Dimethylsulfoxid z. A. und fügt 10 Equivalente Trifluormethansulfonylmethanphosphonsäurediethylester, der aus dem verfügbaren Alkohol 1-Hydroxymethanphosphonsäurediethylester nach Standardmethoden hergestellt wird, hinzu. Nach Erwärmen der Mischung auf 160° wird nach einigen Stunden aufgearbeitet. Man verdünnt mit Natriumhydrogencarbonatlösung, extrahiert mit Methylenchlorid, trocknet und engt ein. Das Rohprodukt wird über Kieselgel mit Essigester und nachfolgend Ethanol chromatographiert.
Gegebenenfalls wird bei der Durchführung eine Base zur Bindung der Trifluormethansulfonsäure zugesetzt.

Man erhält die neuen Verbindungen
(6-Nitro-7-sulfonamido-2,3-dioxo-1,2,3,4-tetrahydrobenzo[f]chinoxalin-1-yl)-methanphosphonsäurediethylester sowie
(6-Nitro-7-sulfonamido-2,3-dioxo-1,2,3,4-tetrahydrobenzo[f]chinoxalin-4-yl)-methanphosphonsäurediethylester.
In gleicher Art und Weise werden hergestellt:
(6-Nitro-8-sulfonamido-2,3-dioxo-1,2,3,4-tetrahydrobenzo[f]chinoxalin-1-yl)-methanphosphonsäurediethylester und
(6-Nitro-8-sulfonamido-2,3-dioxo-1,2,3,4-tetrahydrobenzo[f]chinoxalin-4-yl)-methanphosphonsäurediethylester
(6-Nitro-8,10-disulfonamido-2,3-dioxo-1,2,3,4-tetrahydrobenzo[f]chinoxalin-1-yl)-methanphosphonsäurediethylester und (6-Nitro-8,10-disulfonamido-2,3-dioxo-1,2,3,4-tetrahydrobenzo[f]chinoxalin-4-yl)-methanphosphonsäurediethylester
(6-Nitro-7-sulfonylmethano-2,3-dioxo-1,2,3,4-tetrahydrobenzo[f]chinoxalin-1-yl)-methanphosphonsäurediethylester und (6-Nitro-7-sulfonylmethano-2,3-dioxo-1,2,3,4-tetrahydrobenzo[f]chinoxalin-4-yl)-methanphosphonsäurediethylester
(6-Nitro-7-cyano-2,3-dioxo-1,2,3,4-tetrahydrobenzo[f]chinoxalin-1-yl)-methanphosphonsäurediethylester und
(6-Nitro-7-cyano-2,3-dioxo-1,2,3,4-tetrahydrobenzo[f]chinoxalin-4-yl)-methanphosphonsäurediethylester
(6-Nitro-8-cyano-2,3-dioxo-1,2,3,4-tetrahydrobenzo[f]chinoxalin-1-yl)-methanphosphonsäurediethylester und (6-Nitro-8-cyano-2,3-dioxo-1,2,3,4-tetrahydrobenzo[f]chinoxalin-4-yl)-methanphosphonsäurediethylester
(6-Nitro-7-bromo-2,3-dioxo-1,2,3,4-tetrahydrobenzo[f]chinoxalin-1-yl)-methanphosphonsäurediethylester und (6-Nitro-7-bromo-2,3-dioxo-1,2,3,4-tetrahydrobenzo[f]chinoxalin-4-yl)-methanphosphonsäurediethylester
(6-Nitro-7-trifluormethyl-2,3-dioxo-1,2,3,4-tetrahydrobenzo[f]chinoxalin-1-yl)-methanphosphonsäurediethylester und (6-Nitro-7-trifluormethyl-2,3-dioxo-1,2,3,4-tetrahydrobenzo[f]chinoxalin-4-yl)-methanphosphonsäurediethylester

### Beispiel 10:

### (6-Nitro-2,3-dioxo-1,2,3,4-tetrahydrobenzo[f]chinoxalin-4-yl)-methanphosphonsäure

Die Synthese von 1-Nitro-2-fluornaphthalin erfolgt wie in der Literatur beschrieben. Die Synthese von 1-Nitro-2-chlornaphthalin erfolgt wie in der Literatur beschrieben. 1-Nitro-2-hydroxynaphthalin kann bezogen oder durch Oxidation von Nitroso-2-hydroxynaphthalin dargestellt werden.

### a) Toluolsulfonsäure-(1-nitronaphth-2-ol)-ester

5 g 1-Nitronaphth-1-ol werden in 30 ml Triethylamin mit 5,54g = 1,1 Equivalenten Toluolsulfonsäurechlorid bei 60 ° gelöst und bei dieser Temperatur gerührt bis zum Verschwinden des Edukts. Die Mischung läßt man abkühlen, saugt ab und wäscht den Niederschlag mit kaltem Ethanol. Das Rohprodukt wird aus heißem Essigester umkristallisiert. Man erhält 9,1g Produkt. NMR(DMSO):2.44 (3H s),7.5 (3H d), 7.8 (5H,m) 8.17 (1H m), 8.34 (1H d).
Auf bekannte Weise entstehen:
Nonafluorbutansulfonsäure-(1-nitronaphth-2-ol)-ester
Trifluormethansulfonsäure-(1-nitronaphth-2-ol)-ester
Methansulfonsäure-(1-nitronaphth-2-ol)-ester

### b)(1-Nitro-2-naphthylamino)-methanphosphonsäurediethylester

450 mg Toluolsulfonsäure-(1-nitronaphth-2-ol)-ester werden in 436 mg Aminomethanphosphonsäurediethylester und 0,6 g Diphenylmethan gelöst und 72 Stunden bei 40 - 60° gerührt. Der Ansatz wird mit Methylenchlorid verdünnt, mit 1-n NaOH und zweimal mit Sole gewaschen, getrocknet und einrotiert. Das Rohprodukt wird mit Hexan und Essigester 1:1 als Laufmittel über Kieselgel chromatographiert. Man erhält neben 60% Edukt 31% Produkt als zähes, dunkles Öl.
In gleicher Art und Weise werden hergestellt:
1-Phenyl-1-(1-Nitro-2-naphthylamino)-methanphosphonsäurediethylester
1-(1-Nitro-2-naphthylamino)-ethan-1-phosphonsäurediethylester
1-(1-Nitro-2-naphthylamino)-propan-1-phosphonsäurediethylester
1-(1-Nitro-2-naphthylamino)-butan-1-phosphonsäurediethylester

### c)(1-Amino-2-naphthylamino)-methanphosphonsäurediethylester

170 mg (1-Nitro-2-naphthylamino)-methanphosphonsäurediethylester werden in 2 ml Eisessig mit 10 Equivalenten Eisenpulver reduziert. Nach 2 Stunden wird die Mischung zäh und schließlich fest. Man verdünnt mit Essigester, entfernt das Eisen und chromatographiert mit Essigester. Das Produkt erhält man in 60% Ausbeute.
In gleicher Art und Weise werden hergestellt:
1-Phenyl-1-(1-amino-2-naphthylamino)-methanphosphonsäurediethylester
1-(1-Amino-2-naphthylamino)-ethan-1-phosphonsäurediethylester
1-(1-Amino-2-naphthylamino)-propan-1-phosphonsäurediethylester
1-(1-Amino-2-naphthylamino)-butan-1-phosphonsäurediethylester

### d) Wie unter Beispiel 1 beschrieben cyclisiert man die Amine mit Oxalsäurederivaten zu

1-Phenyl-1-(2,3-dioxo-1,2,3,4-tetrahydrobenzo[f]chinoxalin-4-yl)-methanphosphonsäurediethylester
1-(2,3-dioxo-1,2,3,4-tetrahydrobenzo[f]chinoxalin-4-yl)-ethan-1-phosphonsäurediethylester
1-(2,3-dioxo-1,2,3,4-tetrahydrobenzo[f]chinoxalin-4-yl)-methanphosphonsäurediethylester
1-(2,3-dioxo-1,2,3,4-tetrahydrobenzo[f]chinoxalin-4-yl)-propan-1-phosphonsäurediethylester
1-(2,3-dioxo-1,2,3,4-tetrahydrobenzo[f]chinoxalin-4-yl)-butan-1-phosphonsäurediethylester

### e) Nitrierung der Phosphonate führt zu

1-(6-Nitro-2,3-dioxo-1,2,3,4-tetrahydrobenzo[f]chinoxalin-4-yl)-ethan-1-phosphonsäurediethylester vom Schmelzpunkt >300° (Zers.)
1-(6-Nitro-2,3-dioxo-1,2,3,4-tetrahydrobenzo[f]chinoxalin-4-yl)-methanphosphonsäurediethylester vom Schmelzpunkt 243° C.
1-(6-Nitro-2,3-dioxo-1,2,3,4-tetrahydrobenzo[f]chinoxalin-4-yl)-propan-1-phosphonsäurediethylester
1-(6-Nitro-2,3-dioxo-1,2,3,4-tetrahydrobenzo[f]chinoxalin-4-yl)-butan-1-phosphonsäurediethylester
1-Phenyl-1-(6-nitro-2,3-dioxo-1,2,3,4-tetrahydrobenzo[f]chinoxalin-4-yl)-methanphosphonsäurediethylester

### f) Verseifung zu Phosphonsäuren analog Beispiel 1e).

Man erhält
1-(6-Nitro-2,3-dioxo-1,2,3,4-tetrahydrobenzo[f]chinoxalin-4-yl)-ethan-1-phosphonsäure vom Schmelzpunkt >300° (Zers.)NMR(DMSO): 1.7 (3H d) 5.85(1H), 7.8 (2H m), 8.5 , 8.8, 9.4 je 1H
1-(6-Nitro-2,3-dioxo-1,2,3,4-tetrahydrobenzo[f]chinoxalin-4-yl)-methanphosphonsäure. NMR(DMSO): 4.7(2H d), 7.8 (2H m), 8.5, 8.7, 8.8 je 1H
1-(6-Nitro-2,3-dioxo-1,2,3,4-tetrahydrobenzo[f]chinoxalin-4-yl)-propan-1-phosphonsäure vom Schmelzpunkt >300° (Zers.)
1-(6-Nitro-2,3-dioxo-1,2,3,4-tetrahydrobenzo[f]chinoxalin-4-yl)-butan-1-phosphonsäure1-Phenyl-1-(6-nitro-2,3-dioxo-1,2,3,4-tetrahydrobenzo[f]chinoxalin-4-yl)-methanphosphonsäure

### Beispiel 11:

1-(6-Sulfonamido-2,3-dioxo-1,2,3,4-tetrahydrobenzo[f]chinoxalin-4-yl)-methanphosphonsäure wird nach weiter oben beschriebenen Verfahren aus 4-Amino-3-hydroxy-naphthalin-1-sulfonsäure hergestellt. Analog wird 1-(6-Sulfonamido-2,3-dioxo-1,2,3,4-tetrahydrobenzo[f]chinoxalin-4-yl)-ethan-1-phosphonsäure synthetisiert.

### Beispiel 12:

1-(8-Sulfonamido-2,3-dioxo-1,2,3,4-tetrahydrobenzo[f]chinoxalin-4-yl)-methanphosphonsäure wird nach weiter oben beschriebenen Verfahren aus 6-Hydroxynaphthalin-2-sulfonsäuresalz hergestellt. Analog wird 1-(8-Sulfonamido-2,3-dioxo1,2,3,4-tetrahydrobenzo[f]chinoxalin-4-yl)-ethan-1-phosphonsäure synthetisiert.

### Beispiel 13:

Auf analoge Art und Weise und mit den für Aminosäuren üblichen Methoden stellt man unter Verwendung von handelsüblichen Aminosäuren und deren Derivaten, zum Beispiel tert-Butylestern, beispielsweise folgende Verbindungen dar:
(6-Nitro-2,3-dioxo-1,2,3,4-tetrahydrobenzo[f]chinoxalin-1-yl)-methancarbonsäure
(6-Nitro-7-sulfamoyl-2,3-dioxo-1,2,3,4-tetrahydrobenzo[f]chinoxalin-1-yl)-methancarbonsäure
(7-Sulfamoyl-2,3-dioxo-1,2,3,4-tetrahydrobenzo[f]chinoxalin-1-yl)-methancarbonsäure
(6-Nitro-2,3-dioxo-1,2,3,4-tetrahydrobenzo[f]chinoxalin-4-yl)-methancarbonsäure
1-(6-Nitro-2,3-dioxo-1,2,3,4-tetrahydrobenzo[f]chinoxalin-1-yl)-ethan-1-carbonsäure
1-(6-Nitro-7-sulfamoyl-2,3-dioxo-1,2,3,4-tetrahydrobenzo[f]chinoxalin-1-yl)-ethan-1-carbonsäure
1-(7-Sulfamoyl-2,3-dioxo-1,2,3,4-tetrahydrobenzo[f]chinoxalin-1-yl)-ethan-1-carbonsäure
1-(6-Nitro-2,3-dioxo-1,2,3,4-tetrahydrobenzo[f]chinoxalin-4-yl)-ethan-1-carbonsäure

### Beispiel 14:

1-(6-Nitro-2,3-dioxo-1,2,3,4-tetrahydrobenzo[f]chinoxalin-4-yl)-acetonitril

### a) 1-Nitro-2-naphthylamino-acetonitril.

Zu 21,58 g (80,7 mmol) Methansulfonsäure-(1-nitronaphth-2-ol)-ester werden 32,1 g nach bekannten Vorschriften hergestelltes 1-Aminoacetonitril gegeben. Man rührt 8 Stunden bei 60°, verdünnt dann mit Dichlormethan und zieht die Mischung auf eine mit Kieselgel gefüllte Säule auf. Chromatographie mit Toluol liefert neben 7 g 1-Nitronaphth-2-ol 13% der Theorie an (1-Nitro-2-naphthylamino)-acetonitril.
In gleicher Weise erhält man :
(1 -Nitro-2-naphthylamino)-methyl-acetonitril.
(1 -Nitro-2-naphthylamino)-1,1-dimethyl-acetonitril.

### b) (1-Amino-2-naphthyl)aminoacetonitril.

0,45 g 1-Nitro-2-naphthylamino-acetonitril werden in 65 ml Dioxan werden über Pd/Bariumsulfat als Katalysator bei RT hydriert. Nach Entfernen des Katalysators erhält man 100% Rohprodukt von (1-Amino-2-naphthyl)aminoacetonitril.
Analog werden erhalten:
(1-Amino-2-naphthylamino)-methyl-acetonitril.
(1-Amino-2-naphthylamino)-1,1-dimethyl-acetonitril.

### c) 1-(2,3-Dioxo-1,2,3,4-tetrahydrobenzo[f]chinoxalin-4-yl)-acetonitril

0,41 g Rohprodukt von (1-Amino-2-naphthyl)aminoacetonitril werden analog Beispiel 1d) zu 0,126 g 1-(2,3-Dioxo-1,2,3,4-tetrahydrobenzol[f]chinoxalin-4-yl)-acetonitril umgesetzt. Der Schmelzpunkt ist >350°. NMR(DMSO): 5.4 (2H), 7.55(2H), 7.75, 7.89, 8.03, 8.63 je (1H d), 12.24 (1H s).

### Analog erhält man:

1-(2,3-Dioxo-1,2,3,4-tetrahydrobenzo[f]chinoxalin-4-yl)-methylacetonitril 1-(2,3-Dioxo-1,2,3,4-tetrahydrobenzo[f]chinoxalin-4-yl)-dimethyl-acetonitril

### d) 1-(6-Nitro-2,3-dioxo-1,2,3,4-tetrahydrobenzo[f]chinoxalin-4-yl)-acetonitril

1-(2,3-Dioxo-1,2,3,4-tetrahydrobenzo[f]chinoxalin-4-yl)-acetonitril wird mit 65%iger Salpetersäure 3 Stunden bei RT umgesetzt. Man fällt aus Wasser aus, wäscht die gelben Kristalle und erhält 51% Produkt vom Schmelzpunkt >300° (langsame Zersetzung).

### Analog erhält man:

1-(6 Nitro-2,3-dioxo-1,2,3,4-tetrahydrobenzo[f]chinoxalin-4-yl)-methylacetonitril 1-(6-Nitro-2,3-dioxo-1,2,3,4-tetrahydrobenzo[f]chinoxalin-4-yl)-dimethyl-acetonitril

### Beispiel 15:

1-(6-Nitro-2,3-dioxo-1,2,3,4-tetrahydrobenzo[f]chinoxalin-4-yl)-methantetrazol und
1-(2,3-Dioxo-1,2,3,4-tetrahydrobenzo[f]chinoxalin-4-yl)-methantetrazol

301 mg 1-(2,3-Dioxo-1,2,3,4-tetrahydrobenzo[f]chinoxalin-4-yl)-acetonitril werden in 12 ml N-Methylpyrrolidon mit 3 Equivalenten Natriumazid sowie 1,5 Equivalenten Triethylammoniumchlorid 2,5 Stunden bei 150° gerührt. Nach verdünnen und ansäuern der abgekühlten Mischung werden die Kristalle abgesaugt, mit Wasser und danach Acetonitril gewaschen und getrocknet. Es resultieren 83% 1-(2,3-Dioxo-1,2,3,4-tetrahydrobenzo[f]chinoxalin-4-yl)-methantetrazol vom Schmelzpunkt 320°.
1-(6-Nitro-2,3-dioxo-1,2,3,4-tetrahydrobenzo[f]chinoxalin-4-yl)-methantetrazol wird analog zu Beispiel 14d) aus 1-(2,3-Dioxo-1,2,3,4-tetrahydrobenzo[f]chinoxalin-4-yl)-methantetrazol dargestellt. Fp. >300°.

### Beispiel 16:

### 1-(2,3-Dioxo-1,2,3,4-tetrahydrobenzo[f]chinoxalin-4-yl)-methancarbonsäure-amidoxim.

100 mg 1-(2,3-Dioxo-1,2,3,4-tetrahydrobenzo[f]chinoxalin-4-yl)-acetonitril werden mit 2 Equivalenten Hydroxylaminhydrochlorid sowie Natriumhydrogencarbonat in 0,2 ml Wasser sowie wenig Ethanol 4 Stunden am Rückfluß gekocht. Das Produkt fällt nach Verdünnung mit Wasser aus und wird abgesaugt. Ausbeute 62%. NMR (DMSO): 4.9 (2H s), 5.55 (2H s),7.55 (3H m), 7.75,7.95,8.6 je 1H, 9.2 OH, 12.2 (1H breit).

### Beispiel 17:

1-(6-Nitro-2,3-dioxo-1,2,3,4-tetrahydrobenzo[f]chinoxalin-1-yl)-acetonitril und
1-(6-Nitro-2,3-dioxo-1,2,3,4-tetrahydrobenzo[f]chinoxalin-1-yl)-acetamid
In gleicher Weise wie in Beispiel 14 a) erhält man aus dem entsprechenden Methansulfonsäureester:
2-Nitro-1-naphthylamino-acetonitril
(2-Nitro-1-naphthylamino)-methyl-acetonitril.
(2-Nitro-1-naphthylamino)-1,1-dimethyl-acetonitril.
Analog werden erhalten:
(2-Amino-1-naphthylamino)-methyl-acetonitril.
(2-Amino-1-naphthyl)aminoacetonitril.
(2-Amino-1-naphthylamino)-1,1-dimethyl-acetonitril.
daraus entstehen die Verbindungen
1-(2,3-Dioxo-1,2,3,4-tetrahydrobenzo[f]chinoxalin-1-yl)-acetonitril NMR(DMSO):5.2 (2H), 7.45, 7.53, 7.68, 7.83,8.03, 8.14 je (1H d), 12.2 (1H s).
1 -(2,3-Dioxo-1,2,3,4-tetrahydrobenzo[f]chinoxalin-1-yl)-methylacetonitril
1-(2,3-Dioxo-1,2,3,4-tetrahydrobenzo[f]chinoxalin-1-yl)-dimethyl-acetonitril
1-(6-Nitro-2,3-dioxo-1,2,3,4-tetrahydrobenzo[f]chinoxalin-1-yl)-acetamid:

1-(2,3-Dioxo-1,2,3,4-tetrahydrobenzo[f]chinoxalin-1-yl)-acetonitril wird mit 65%iger Salpetersäure 3 Stunden bei RT umgesetzt. Man fällt aus Wasser aus, wäscht die gelben Kristalle und erhält ca 100% Rohprodukt vom Schmelzpunkt >300° (langsame Zersetzung). Es handelt sich um 1-(6-Nitro-2,3-dioxo-1,2,3,4-tetrahydrobenzo[f]chinoxalin-1-yl)-acetamid.
1-(6-Nitro-2,3-dioxo-1,2,3,4-tetrahydrobenzo[f]chinoxalin-1-yl)-acetonitril entsteht unter Verwendung von Nitroniumtetrafluorborat und schmilzt >300°.
Analog erhält man:
1-(6 Nitro-2,3-dioxo-1,2,3,4-tetrahydrobenzo[f]chinoxalin-1-yl)-methylacetonitril
1-(6-Nitro-2,3-dioxo-1,2,3,4-tetrahydrobenzo[f]chinoxalin-1-yl)-dimethyl-acetonitril

### Beispiel 18:

1-(6-Nitro-2,3-dioxo-1,2,3,4-tetrahydrobenzo[f]chinoxalin-1-yl)-methantetrazol (Fp. 305-315° Zers.) und
1-(2,3-Dioxo-1,2,3,4-tetrahydrobenzo[f]chinoxalin-1-yl)-methantetrazol (Fp. ca. 310°Zers.)
werden wie in Beispiel 15 beschrieben dargestellt .

### Beispiel 19:

### 1-(2,3-Dioxo-1,2,3,4-tetrahydrobenzo[f]chinoxalin-1-yl)-methancarbonsäure-amidoxim.

100 mg 1-(2,3-Dioxo-1,2,3,4-tetrahydrobenzo[f]chinoxalin-1-yl)-acetonitril werden mit 2 Equivalenten Hydroxylaminhydrochlorid sowie Natriumhydrogencarbonat in 0,2 ml Wasser sowie wenig Ethanol 7 Stunden am Rückfluß gekocht. Das Produkt fällt nach Verdünnung mit Wasser aus und wird abgesaugt. Ausbeute 67%. NMR (DMSO): 4.9 (2H s),5.7 (2H s),7.5 (3H m), 7.75,7.95,8.4 je 1H, 9.12 OH, 12.14 (1H breit).

### Beispiel 20

### [6-(Piperidin-1-yl)-2,3-dioxo-1,2,3,4-tetrahydrobenzo[f]chinoxalin-4-yl]-methanphosphonsäurediethylester

Zu 37 mg (6-Amino-2,3-dioxo-1,2,3,4-tetrahydrobenzo[f]chinoxalin-4-yl)-methanphosphonsäurediethylester in 2 ml Tetrahydrofuran z.A. ,zu dem 13 mg Natriumborhydrid gefügt wurden, gibt man unter Rühren bei Eisbadtemperatur eine Mischung von 100 Mikrolitern wässrigem Glutardialdehyd (Gehalt ca. 25%ig), 300 Mikrolitern verdünnter Schwefelsäure und 1 ml Tetrahydrofuran/Methanol 1:1 .Dann fügt man weitere 10 mg Natriumboranat hinzu und arbeitet nach 1 Stunde auf. Die Mischung wird neutralisiert und mit Ethylacetat extrahiert. Man erhält nach Chromatographie mit wenig Kieselgel 16 mg Produkt.
Nach Verseifung des Esters erhält man

### [6-(Piperidin-1-yl)-2,3-dioxo-1,2,3,4-tetrahydrobenzo[f]chinoxalin-4-yl]-methanphosphonsäure.

Analog synthetisiert man
[6-(Piperidin-1-yl)-2,3-dioxo-1,2,3,4-tetrahydrobenzo[f]chinoxalin-1-yl]-methanphosphonsäurediethylester sowie
[6-(Piperidin-1-yl)-2,3-dioxo-1,2,3,4-tetrahydrobenzo[f]chinoxalin-1-yl]-methanphosphonsäure.

### Beispiel 21

### [6-(Morpholin-1-yl)-2,3-dioxo-1,2,3,4-tetrahydrobenzo[f]chinoxalin-4-yl]-methanphosphonsäure

[6-(Morpholin-1-yl)-2,3-dioxo-1,2,3,4-tetrahydrobenzo[f]chinoxalin-4-yl]-methanphosphonsäurediethylester wird aus (6-Amino-2,3-dioxo-1,2,3,4-tetrahydrobenzo[f]chinoxalin-4-yl)-methanphosphonsäurediethylester nach der in Beispiel 20 beschriebenen Methode durch Umsetzung mit 3-Oxa-glutarsäuredialdehyd erhalten. Man gelangt auf üblichem Weg zu
[6-(Morpholin-1-yl)-2,3-dioxo-1,2,3,4-tetrahydrobenzo[f]chinoxalin-4-yl]-methanphosphonsäure.
Analog werden erhalten:
[6-(Morpholin-1-yl)-2,3-dioxo-1,2,3,4-tetrahydrobenzo[f]chinoxalin-1-yl]-methanphosphonsäurediethylester sowie
[6-(Morpholin-1-yl)-2,3-dioxo-1,2,3,4-tetrahydrobenzo[f]chinoxalin-1-yl]-methanphosphonsäure. und
1-[6-(Morpholin-1-yl)-2,3-dioxo-1,2,3,4-tetrahydrobenzo[f]chinoxalin-1-yl]-ethanphosphonsäurediethylester sowie
1-[6-(Morpholin-1-yl)-2,3-dioxo-1,2,3,4-tetrahydrobenzo[f]chinoxalin-1-yl]-ethanphosphonsäure.

### Beispiel 22

1-[6-(Imidazolyl)-2,3-dioxo-1,2,3,4-tetrahydrobenzo[f]chinoxalin-1-yl]-ethanphosphonsäurediethylester beziehungsweise 1-[6-(Imidazolyl)-2,3-dioxo-1,2,3,4-tetrahydrobenzo[f]chinoxalin-1-yl]-ethanphosphonsäure werden durch nucleophile aromatische Substitution aus den Halogenderivaten mit Imidazol als Nucleophil dargestellt. Ebenfalls werden dargestellt
[6-(Imidazolyl)-2,3-dioxo-1,2,3,4-tetrahydrobenzo[f]chinoxalin-1-yl]-methanphosphonsäurediethylester
[6-(Imidazolyl)-2,3-dioxo-1,2,3,4-tetrahydrobenzo[f]chinoxalin-1-yl]-methanphosphonsäure
[6-(Imidazolyl)-2,3-dioxo-1,2,3,4-tetrahydrobenzo[f]chinoxalin-4-yl]-methanphosphonsäurediethylester
[6-(Imidazolyl)-2,3-dioxo-1,2,3,4-tetrahydrobenzo[f]chinoxalin-4-yl]-methanphosphonsäure.

## Patentansprüche

1. Benzo[f]chinoxalindionderivate der Formel I 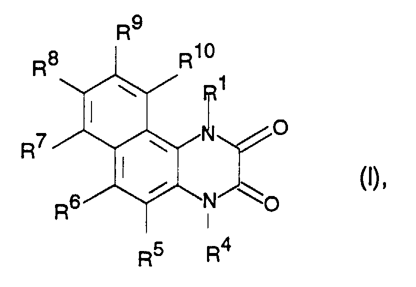 worin
R¹ und R⁴ gleich oder verschieden sind und Wasserstoff, mit R² substituiertes C₁₋₁₂-Alkyl, mit R² substituiertes C₂₋₁₂-Alkenyl, mit R² substituiertes C₂₋₁₂-Alkinyl, mit R² substituiertes C₃₋₇-Cycloalkyl, -(CH₂)ₙ-C₆₋₁₂-Aryl, das im Aryl- oder im Alkylrest mit R² substituiert ist oder -(CH₂)ₙ- Hetaryl, das im Hetaryl- oder Alkylrest mit R² substituiert ist und R¹ und R⁴ nicht gleichzeitig Wasserstoff bedeuten,
R⁵, R⁶, R⁷, R⁸, R⁹ und R¹⁰ gleich oder verschieden sind und Wasserstoff, Halogen, Nitro, NR¹⁶R¹⁷, NHCOR¹¹, SO₀₋₃R¹², C₃₋₇-Cycloalkyloxy, COR¹³, Cyano, CF₃, OCH₂CF₃,C₁₋₆- Alkyl oder C₁₋₄-Alkoxy,
wobei
R² -CN,-Tetrazol,-C(NOH)NH2,-CO-R³ oder -PO-XY und R² ein- bis zweifach gleich oder verschieden steht und
n 0, 1, 2, 3, 4 oder 5 ist,
R³ Hydroxy, C₁₋₆-Alkoxy oder NR¹⁴R¹⁵,
X und Y gleich oder verschieden sind und Hydroxy, C₁₋₆-Alkoxy, -O-(CH₂)ₚ-O-, C₁₋₄-Alkyl oder NR¹⁴R¹⁵ bedeuten und p 1, 2 oder 3 ist und
R¹¹ C₁₋₆-Alkyl oder Phenyl, das mit Halogen substituiert sein kann,
R¹² Wasserstoff, C₁₋₄-Alkyl, NH₂, N(C₁₋₄-Alkyl)₂, -NH(C₁₋₄-Alkyl), -NH-CH₂CONH₂, -CH₂CONH₂, CF₃ oder -NH-(CH₂)ₙ-R² und
R¹³ Hydroxy, C₁₋₆-Alkoxy, C₁₋₆-Alkyl oder NR¹⁴R¹⁵ bedeuten
R¹⁴ und R¹⁵ , R¹⁶ und R¹⁷ gleich oder verschieden sind und Wasserstoff, -CO-C₁₋₆-Alkyl, Phenyl oder C₁₋₆-Alkyl, das gegebenenfalls mit C₁₋₄-Alkoxy oder einer gegebenenfalls mit C₁₋₄-Alkyl mono- oder di-substituierter Aminogruppe substituiert sein kann, oder gemeinsam mit dem Stickstoffatom einen 5-7-gliedrigen gesättigten Heterocyclus bilden, der ein weiteres N-, S- oder O-Atom enthalten und substituiert sein kann oder einen ungesättigten 5-gliedrigen Heterocyclus bilden, der 1-3 N-Atome enthalten und substituiert sein kann,
sowie deren Isomeren oder Salze, wobei, falls R⁵-R¹⁰ Wasserstoff ist, R¹ oder R⁴ nicht Methanphosphonsäure oder Ethan-1-phosphonsäure bedeuten.

2. (6-Nitro-2,3-dioxo-1,2,3,4-tetrahydrobenzo[f]chinoxalin-1-yl)-methanphosphonsäure
(6-Nitro-7-sulfamoyl-2,3-dioxo-1,2,3,4-tetrahydrobenzo[f]chinoxalin-1-yl)-methanphosphonsäure
(6-Nitro-7-sulfonamido-2,3-dioxo-1,2,3,4-tetrahydrobenzo[f]chinoxalin-4-yl)-methanphosphonsäure
(6-Nitro-8-sulfonamido-2,3-dioxo-1,2,3,4-tetrahydrobenzo[f]chinoxalin-1-yl)-methanphosphonsäure
(6-Nitro-8-sulfonamido-2,3-dioxo-1,2,3,4-tetrahydrobenzo[f]chinoxalin-4-yl)-methanphosphonsäure
(6-Nitro-8,10-disulfonamido-2,3-dioxo-1,2,3,4-tetrahydrobenzo[f]chinoxalin-1-yl)-methanphosphonsäure
(6-Nitro-8,10-disulfonamido-2,3-dioxo-1,2,3,4-tetrahydrobenzo[f]chinoxalin-4-yl)-methanphosphonsäure
(6-Nitro-2,3-dioxo-1,2,3,4-tetrahydrobenzo[f]chinoxalin-1-yl)-methancarbonsäure
(6-Nitro-7-sulfamoyl-2,3-dioxo-1,2,3,4-tetrahydrobenzo[f]chinoxalin-1-yl)-methancarbonsäure
(6-Nitro-2,3-dioxo-1,2,3,4-tetrahydrobenzo[f]chinoxalin-4-yl)-methancarbonsäure
1-(6-Nitro-2,3-dioxo-1,2,3,4-tetrahydrobenzo[f]chinoxalin-1-yl)-ethan-1-carbonsäure
1-(6-Nitro-7-sulfamoyl-2,3-dioxo-1,2,3,4-tetrahydrobenzo[f]chinoxalin-1-yl)-ethan-1-carbonsäure
1-(6-Nitro-2,3-dioxo-1,2,3,4-tetrahydrobenzo[f]chinoxalin-4-yl)-ethan-1-carbonsäure
(6-Nitro-2,3-dioxo-1,2,3,4-tetrahydrobenzo[f]chinoxalin-4-yl)-methanphosphonsäure

3. Arzneimittel auf Basis der Verbindungen nach Anspruch 1 und 2.

4. Verfahren zur Herstellung der Verbindungen der Formel I nach Anspruch 1, dadurch gekennzeichnet, daß man
a) eine Verbindung der Formel II oder III 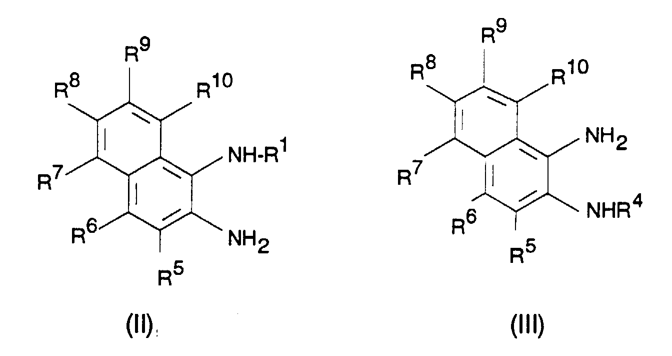 worin R¹ bis R¹⁰ die obige Bedeutung haben mit Oxalsäure oder reaktiven Oxalsäurederivaten cyclisiert oder
b) eine Verbindung der Formel IV oder V 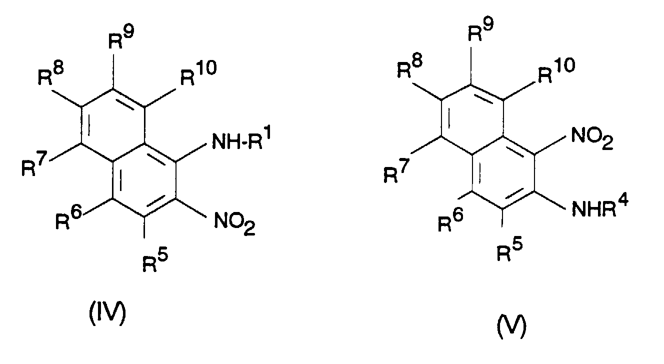 worin R¹ bis R¹⁰ die obige Bedeutung haben mit Oxalsäure oder reaktiven Oxalsäurederivaten umsetzt und nach Reduktion der Nitrogruppe cyclisiert oder
c) eine Verbindung der Formel VI 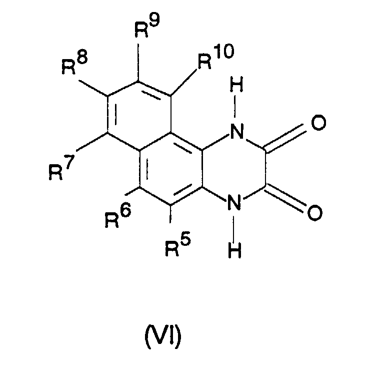 worin R⁵ bis R¹⁰ die obige Bedeutung haben in Gegenwart einer Base mit R¹ Z oder R⁴ Z, wobei Z eine Fluchtgruppe darstellt, umsetzt und gewünschtenfalls anschließend die Estergruppe verseift oder die Säuregruppe verestert oder amidiert oder die Nitrogruppe reduziert zur Aminogruppe oder die Aminogruppe alkyliert oder acyliert oder die Aminogruppe gegen Halogen oder Cyano austauscht oder eine Nitrogruppe oder Halogen einführt oder ein Nitril in das Tetrazol oder Amidoxim überführt oder nucleophil substituiert oder die Isomeren trennt oder die Salze bildet.

## Claims

1. Benzo[f]quinoxalinedione derivatives of formula I 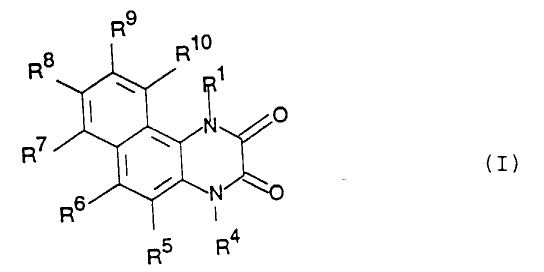 wherein
R¹ and R⁴ are identical or different and represent hydrogen, R²-substituted C₁₋₁₂-alkyl, R²-substituted C₂₋₁₂-alkenyl, R²-substituted C₂₋₁₂-alkynyl, R²-substituted C₃₋₇-cycloalkyl, -(CH₂)ₙ-C₆₋₁₂-aryl, which is substituted in the aryl or alkyl moiety by R², or -(CH₂)ₙ-hetaryl, which is substituted in the hetaryl or alkyl moiety by R², and R¹ and R⁴ do not simultaneously represent hydrogen.
R⁵, R⁶, R⁷, R⁸, R⁹ and R¹⁰ are identical or different and represent hydrogen, halogen, nitro, NR¹⁶R¹⁷, NHCOR¹¹, SO₀₋₃R¹², C₃₋₇-cycloalkyloxy, COR¹³, cyano, CF₃, OCH₂CF₃, C₁₋₆-alkyl or C₁₋₄-alkoxy,
wherein
R² represents -CN, -tetrazole, -C(NOH)NH₂, -CO-R³ or -PO-XY and R₂ is present once or twice, the same or different, and
n is 0, 1, 2, 3, 4 or 5,
R³ represents hydroxy, C₁₋₆-alkoxy or NR¹⁴R¹⁵,
X and Y are identical or different and represent hydroxy, C₁₋₆-alkoxy, -O-(CH₂)ₚ-O-, C₁₋₄-alkyl or NR¹⁴R¹⁵, and p is 1, 2 or 3, and
R¹¹ represents C₁₋₆-alkyl or phenyl, which may be substituted by halogen,
R¹² represents hydrogen, C₁₋₄-alkyl, NH₂, N(C₁₋₄-alkyl)₂, -NH(C₁₋₄-alkyl), -NH-CH₂CONH₂, -CH₂CONH₂, CF₃ or -NH-(CH₂)ₙ-R² and
R¹³ represents hydroxy, C₁₋₆-alkoxy, C₁₋₆-alkyl or NR¹⁴R¹⁵,
R¹⁴ and R¹⁵, R¹⁶ and R¹⁷ are identical or different and represent hydrogen, -CO-C₁₋₆-alkyl, phenyl or C₁₋₆-alkyl, which may optionally be substituted by C₁₋₄-alkoxy or by an amino group optionally mono- or di-substituted by C₁₋₄-alkyl, or together with the nitrogen atom form a 5- to 7-membered saturated heterocycle that may contain a further N, S or O atom and may be substituted, or form an unsaturated 5-membered heterocycle that may contain from 1 to 3 N atoms and may be substituted,
and also the isomers or salts thereof, wherein if R⁵-R¹⁰ is hydrogen, neither R¹ nor R⁴ can represent methane-phosphonic acid or ethane-1-phosphonic acid.

2. (6-Nitro-2,3-dioxo-1,2,3,4-tetrahydrobenzo[f]quinoxalin-1-yl)-methanephosphonic acid
(6-nitro-7-sulphamoyl-2,3-dioxo-1,2,3,4-tetrahydrobenzo[f]quinoxalin-1-yl)-methanephosphonic acid
(6-nitro-7-sulphonamido-2,3-dioxo-1,2,3,4-tetrahydrobenzo[f]quinoxalin-4-yl)-methanephosphonic acid
(6-nitro-8-sulphonamido-2,3-dioxo-1,2,3,4-tetrahydrobenzo[f]quinoxalin-1-yl)-methanephosphonic acid
(6-nitro-8-sulphonamido-2,3-dioxo-1,2,3,4-tetrahydrobenzo[f]quinoxalin-4-yl)-methanephosphonic acid
(6-nitro-8,10-disulphonamido-2,3-dioxo-1,2,3,4-tetrahydrobenzo[f]quinoxalin-1-yl)-methanephosphonic acid
(6-nitro-8,10-disulphonamido-2,3-dioxo-1,2,3,4-tetrahydrobenzo[f]quinoxalin-4-yl)-methanephosphonic acid
(6-nitro-2,3-dioxo-1,2,3,4-tetrahydrobenzo[f]quinoxalin-1-yl)-methanecarboxylic acid
(6-nitro-7-sulphamoyl-2,3-dioxo-1,2,3,4-tetrahydrobenzo[f]quinoxalin-1-yl)-methanecarboxylic acid
(6-nitro-2,3-dioxo-1,2,3,4-tetrahydrobenzo[f]quinoxalin-4-yl)-methanecarboxylic acid
1-(6-nitro-2,3-dioxo-1,2,3,4-tetrahydrobenzo[f]quinoxalin-1-yl)-ethane-1-carboxylic acid
1-(6-nitro-7-sulphamoyl-2,3-dioxo-1,2,3,4-tetrahydrobenzo[f]quinoxalin-1-yl)-ethane-1-carboxylic acid
1-(6-nitro-2,3-dioxo-1,2,3,4-tetrahydrobenzo[f]quinoxalin-4-yl)-ethane-1-carboxylic acid
(6-nitro-2,3-dioxo-1,2,3,4-tetrahydrobenzo[f]quinoxalin-4-yl)-methanephosphonic acid

3. Medicament based on the compounds according to claims 1 and 2.

4. Process for the preparation of compounds of formula I according to claim 1, characterised in that
a) a compound of formula II or III 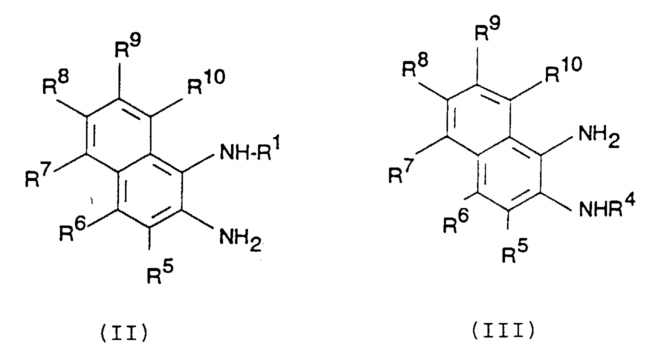 wherein R¹ to R¹⁰ have the above meanings is cyclised with oxalic acid or reactive oxalic acid derivatives or
b) a compound of formula IV or V 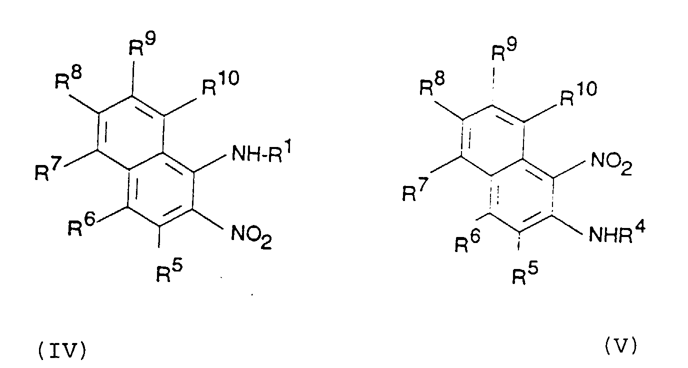 wherein R¹ to R¹⁰ have the above meanings is reacted with oxalic acid or reactive oxalic acid derivatives and, after reduction of the nitro group, is cyclised, or
c) a compound of formula VI 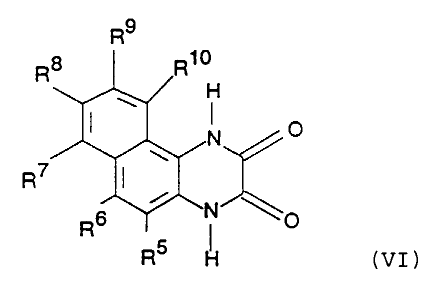 wherein R⁵ to R¹⁰ have the above meanings is reacted in the presence of a base with R¹Z or R⁴Z, wherein Z is a leaving group, and, if desired, then the ester group is hydrolysed or the acid group is esterified or amidated or the nitro group is reduced to the amino group or the amino group is alkylated or acylated or the amino group is replaced by halogen or by cyano or a nitro group or halogen is introduced or a nitrile is converted into the tetrazole or amidoxime or nucleophilically substituted or the isomers are separated or salts are formed.

## Revendications

1. Dérivés de benzo[f]quinoxalindione de formule I 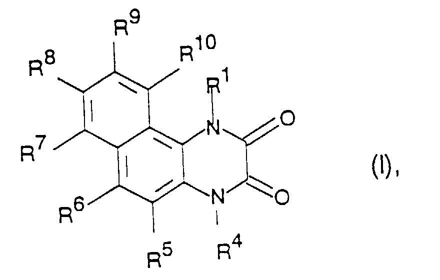 dans laquelle R¹ et R⁴ sont identiques ou différents et représentent l'hydrogène, alkyle en C₁-C₁₂ substitué par R², alcényle en C₂-C₁₂ substitué par R², alcynyle en C₂-C₁₂ substitué par R², cycloalkyle en C₃-C₇ substitué par R², -(CH₂)ₙ-aryle en C₆-C₁₂, lequel est substitué dans le radical aryle ou alkyle par R² ou -(CH₂)ₙ-hétaryle, lequel est substitué dans le radical hétaryle ou alkyle par R², et R¹ et R⁴ ne représentent pas simultanément l'hydrogène,
R⁵, R⁶, R⁷, R⁸, R⁹ et R¹⁰ sont identiques ou différents et représentent l'hydrogène, halogène, nitro, NR¹⁶R¹⁷, NHCOR¹¹, SO₀₋₃R¹², cycloalkyloxy en C₃-C₇, COR¹³, cyano, CF₃, OCH₂CF₃, alkyle en C₁-C₆ ou alcoxy en C₁-C₄,
R² étant -CN, -tétrazol, -C(NOH)NH₂, -CO-R³ ou -PO-XY et R² étant une fois ou deux fois identique ou différent et n vaut 0, 1, 2, 3, 4 ou 5,
R³ représente hydroxy, alcoxy en C₁-C₆ ou NR¹⁴R¹⁵,
X et Y sont identiques ou différents et représentent hydroxy, alcoxy en C₁-C₆, -O-(CH₂)ₚ-O-, alkyle en C₁-C₄ ou NR¹⁴R¹⁵, et p vaut 1, 2 ou 3 et
R¹¹ représente alkyle en C₁-C₆ ou phényle qui peut être substitué par halogène,
R¹² représente l'hydrogène, alkyle en C₁-C₄, NH₂, N(alkyle en C₁-C₄)₂, -NH(alkyle en C₁-C₄), -NH-CH₂CONH₂, -CH₂CONH₂, CF₃ ou -NH-(CH₂)ₙ-R² et
R¹³ représente hydroxy, alcoxy en C₁-C₆, alkyle en C₁-C₆ ou NR¹⁴R¹⁵,
R¹⁴ et R¹⁵, R¹⁶ et R¹⁷ sont identiques ou différents et représentent l'hydrogène, -CO-alkyle en C₁-C₆, phényle ou alkyle en C₁-C₆, qui peut être éventuellement substitué par alcoxy en C₁-C₄ ou un groupe amino mono- ou disubstitué éventuellement par alkyle en C₁-C₄, ou ensemble avec l'atome d'azote forment un hétérocycle saturé à 5 à 7 chaînons, qui peut être substitué et contenir un autre atome de N, S ou O ou un hétérocycle insaturé à 5 chaînons, qui peut être substitué et contenir 1 à 3 atomes de N,
ainsi que leurs isomères ou sels, dans le cas où R⁵-R¹⁰ est l'hydrogène, R¹ ou R⁴ ne représente pas l'acide méthane-phosphonique ou l'acide éthane-1-phosphonique.

2. Acide (6-nitro-2,3-dioxo-1,2,3,4-tétrahydrobenzo[f]quinoxalin-1-yl)-méthanephosphonique
Acide (6-nitro-7-sulfamoyl-2,3-dioxo-1,2,3,4-tétrahydrobenzo[f]quinoxalin-1-yl)-méthanephosphonique
Acide (6-nitro-7-sulfonamido-2,3-dioxo-1,2,3,4-tétrahydrobenzo[f]quinoxalin-4-yl)-méthanephosphonique
Acide (6-nitro-8-sulfonamido-2,3-dioxo-1,2,3,4-tétrahydrobenzo[f]quinoxalin-1-yl)-méthanephosphonique
Acide (6-nitro-8-sulfonamido-2,3-dioxo-1,2,3,4-tétrahydrobenzo[f]quinoxalin-4-yl)-méthanephosphonique
Acide (6-nitro-8,10-disulfonamido-2,3-dioxo-1,2,3,4-tétrahydrobenzo[f]quinoxalin-1-yl)-méthanephosphonique
Acide (6-nitro-8,10-disulfonamido-2,3-dioxo-1,2,3,4-tétrahydrobenzo[f]quinoxalin-4-yl)-méthanephosphonique
Acide (6-nitro-2,3-dioxo-1,2,3,4-tétrahydrobenzo[f]quinoxalin-1-yl)-méthanecarboxylique
Acide (6-nitro-7-sulfamoyl-2,3-dioxo-1,2,3,4-tétrahydrobenzo[f]quinoxalin-1-yl)-méthanecarboxylique
Acide (6-nitro-2,3-dioxo-1,2,3,4-tétrahydrobenzo[f]quinoxalin-4-yl)-méthanecarboxylique
Acide 1-(6-nitro-2,3-dioxo-1,2,3,4-tétrahydrobenzo[f]quinoxalin-1-yl)-éthane-1-carboxylique
Acide 1-(6-nitro-7-sulfamoyl-2,3-dioxo-1,2,3,4-tétrahydrobenzo[f]quinoxalin-1-yl)-éthane-1-carboxylique
Acide 1-(6-nitro-2,3-dioxo-1,2,3,4-tétrahydrobenzo[f]quinoxalin-4-yl)-éthane-1-carboxylique
Acide (6-nitro-2,3-dioxo-1,2,3,4-tétrahydrobenzo[f]quinoxalin-4-yl)-méthanephosphonique.

3. Médicaments à base des composés selon les revendications 1 et 2.

4. Procédé pour la préparation des composés de formule I selon la revendication 1, caractérisé en ce que
a) par cyclisation d'un composé de formule II ou III 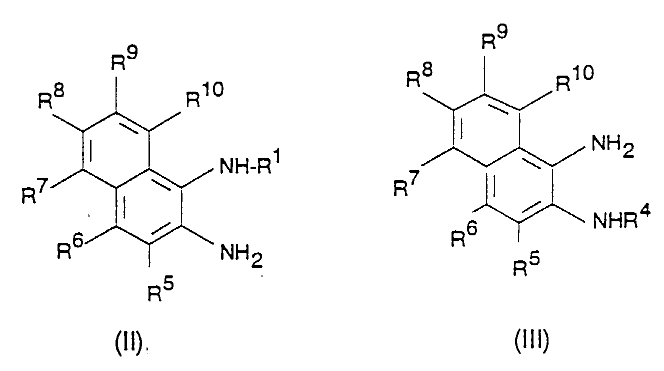 dans lesquelles R¹ à R¹⁰ ont les significations données ci-dessus avec l'acide oxalique ou des dérivés réactifs de l'acide oxalique, ou
b) par réaction d'un composé de formule IV ou V 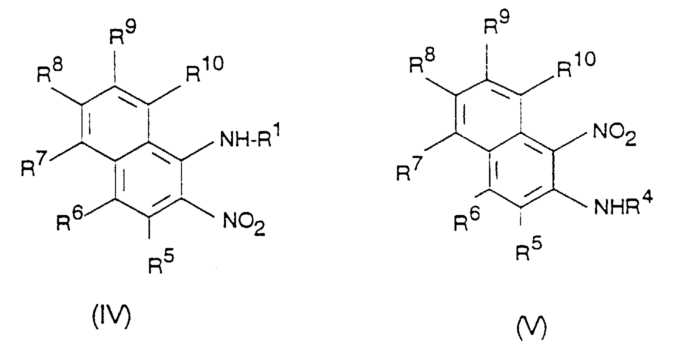 dans lesquelles R¹ à R¹⁰ ont les significations données ci-dessus avec l'acide oxalique ou des dérivés réactifs de l'acide oxalique et, après réduction du groupe nitro, par cyclisation ou
c) par réaction d'un composé de formule VI 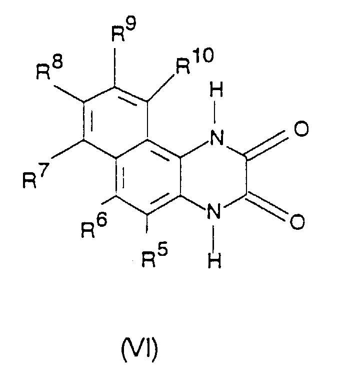 dans laquelle R⁵ à R¹⁰ ont les significations données ci-dessus en présence d'une base avec R¹ représentant Z ou R⁴ représentant Z, Z étant un groupe éliminable, et si on le souhaite ensuite par saponification du groupe ester ou par amidification ou estérification du groupe acyle ou par réduction du groupe nitro en groupe amino ou par alkylation ou acylation du groupe amino, ou par échange du groupe amino contre un halogèno ou un groupe cyano, ou par introduction d'un groupe nitro ou halogéno, ou par substitution nucléophile ou par transformation d'un nitrile en le tétrazole ou l'amidoxime, ou par séparation des isomères ou par formation de sels.
